# EUROPEAN PATENT SPECIFICATION

(11) **EP 3 882 607 B1**
(45) Date of publication and mention of the grant of the patent: **30.07.2025**
(21) Application number: 19883514.2
(22) Date of filing: 08.11.2019
(51) Int. Cl.: G01N 21/84

(54) **INTEGRATED IMMUNODIAGNOSTIC FLUORESCENCE READER HAVING MULTIPLE DIAGNOSES FUNCTION**
INTEGRIERTES IMMUNDIAGNOSTISCHES FLUORESZENZLESEGERÄT MIT MULTIDIAGNOSEFUNKTION
LECTEUR DE FLUORESCENCE D'IMMUNODIAGNOSTIC INTÉGRÉ DOTÉ D'UNE FONCTION DE DIAGNOSTICS MULTIPLES

(30) Priority: 14.11.2018 KR 20180139578
(43) Date of publication of application: 22.09.2021
(73) Proprietor: Boditech Med Inc., Chuncheon-si, Gangwon-do 24398 (KR)
(72) Inventor: NAHM, Kie Bong, Seoul 06717 (KR); CHA, Min Seok, Chuncheon-si Gangwon-do 24415 (KR); CHO, Chu Hyun, Chuncheon-si Gangwon-do 24399 (KR); PARK, Sang Hyun, Seoul 07360 (KR)
(74) Representative: Ter Meer Steinmeister & Partner
(86) International application number: PCT/KR2019/015154
(87) International publication number: WO 2020/101280

(56) References cited:
- WO-A1-2018/043857
- CN-A- 102 087 215
- CN-U- 205 643 162
- JP-A- 2014 071 084
- KR-A- 20050 105 176
- KR-A- 20110 060 439
- KR-A- 20160 047 890
- KR-B1- 101 789 679
- US-A1- 2006 216 832
- US-A1- 2011 053 289
- US-A1- 2013 230 845

## Description

### [Technical Field]

The present disclosure relates to a fluorescence reader used for immunodiagnosis, and more particularly, to a fluorescence reader with a multi-diagnosis function, which is configured to be capable of identifying and classifying various samples through barcodes differently assigned for each diagnosis marker and of adjusting an optimal time required for a later flow of samples.

### [Background Art]

Various diagnostic kits for detecting a specific target material in liquid samples, such as blood, have been developed and conveniently and widely used for rapid diagnosis of a disease. Among these kits, a diagnostic kit based on immunochromatographic assay is widely used to detect the status of disease or to monitor its development. In particular, a representative example is a test based on a lateral flow method.

In such diagnostic kits, a fluorescent material is used as a labeling agent for detecting a target molecule and accordingly, a reader for detecting the signal from the fluorescent material is required. Although such fluorescence readers may be able to perform an accurate detection per se, its complicated structure may result in malfunctions and thus inaccurate results even by the trained user in many cases.

In addition, recently there are emerging multi-diagnostic type devices having high sensitivity and being capable of perform various types of tests compared to a rapid kit which depends on the human eye for reading the results. However, such multi-diagnostic type devices tend to become more complicated to use and complex in structure than a single test device and to have a size larger than the single test device. Also is a problem a high production cost associated with a longer time for manufacturing and quality control. US 2013/230845 A1 relates to a system and an apparatus for analysis of a sample to aid in medical diagnosis or detection of the presence or absence of an analyte in the sample. WO 2018/043857 A1 relates to a fluorescence signal readout apparatus, and more particularly, to a fluorescence signal reading apparatus capable of detecting a flow of a solution analyzed by a lateral flow method and automatically calculating a flow time per actual unit length of a preform.

In a lateral flow method for quantification, one of the factors to increase the accuracy is to scan a signal from a cartridge at an exact predetermined time. In a conventional method, in order to save time to obtain the results from the test using multiple cartridges, a semiautomatic process is used in which samples are loaded into a cartridge outside of a fluorescence reader, and incubated for a certain amount time for reaction to occur, then the cartridge is inserted into the reader, and the button is pressed to start a scan. A problem of this process is that the reaction time may not be optimal as manufacturers intend. The reason for this is that a total measurement time may unintentionally vary and thus be increased or decreased depending on various factors such as the time taken for a user to insert the cartridge at the end of a reaction time, the time taken for the user to press the button, and the time taken for the reader to perform a scan, which may be different case by case or user by user basis. If the total measurement time is increased as expected, the test may become inefficient, or if the total measurement time is decreased, the quality of the test is not optimal. Accordingly, there is a need for development a fluorescence reader capable of overcoming such disadvantages.

Furthermore, in a multi-diagnostic device for diagnosing or testing several types of diseases not a device for diagnosing a single disease, it is also necessary to implement an error prevention mechanism capable of preventing a danger which may occur when a user mistakenly inserts a wrong cartridge for test.

Korean Patent Application Publication No. 10-2015-0029290 relates to a device of a reader for diagnostic strip, and discloses a reader characterized by comprising a stage on which a diagnostic strip having two or more detection areas lengthwise is located, a light source and a light detector disposed on one side of the stage, and means for movement for moving the stage lengthwise, wherein signal can be continuously obtained from the two or more detection areas (reaction area and comparative area) by moving the diagnostic strip by the movement means. However, the device has problems in that a user cannot perform a measurement at an exact time intended and mixing of the cartridges cannot be prevented when the multiple cartridges of different samples are employed.

### [Summary of the Invention]

Aspects of the present invention provide a fluorescence reader used for immunodiagnosis having a multi-diagnosis function and an error prevention function, which can prevent an error by identifying different types of diagnostic cartridges when various diagnostic cartridges are inserted. Aspects of the present invention can simultaneously identify the different type of diagnosis markers or samples, etc. by automatically calculating a reaction time by detecting a sample flowing into a membrane when the sample is loaded. One or more objects are solved by the features of the independent claim.

The present invention provides an integrated fluorescence reader which may be used for immunodiagnosis, comprising: a base frame with an open top surface, comprising: an entrance part at a front side thereof into which a diagnostic cartridge having a barcode differently assigned to each diagnosis marker and a fluorescence measurement window is insertable, wherein the barcode is located on the same side as the fluorescence measurement window; an insertion space opened to an outside through the entrance part, wherein the cartridge is insertable through the entrance part; a space for the movement of an optical module, being located on an upper part of the insertion space in which the optical module is configured to move; a space for driving module mounting, being located on one side of the space for the movement of the optical module; a space for reference position detection module mounting; a cartridge fixing part configured for fixing the diagnostic cartridge at a position by applying pressure to at least part of the diagnostic cartridge when the diagnostic cartridge is inserted into the insertion space; the optical module located in the space for the movement of the optical module of the base frame; a driving module located in the space for driving module mounting of the base frame and connected to the optical module to move the optical module, wherein the driving module includes two guide shafts, each fixed to the base frame in parallel to a lengthwise direction of the diagnostic cartridge located in the insertion space, wherein one end of each of the two guide shafts is fit and fixed in fitting grooves of the base frame and the other end of each of the two guide shafts is mounted in mounting grooves of the base frame, wherein the optical module is configured to move along the two guide shafts and to radiate light to the fluorescence measurement window and the barcode of the diagnostic cartridge while moving in parallel to the lengthwise direction of the diagnostic cartridge along the two guide shafts and to detect fluorescence light and reflected light reflected from the barcode; a detection module for a reference position located in the space for reference position detection module mounting of the base frame and configured to detect a reference position of the optical module and to detect whether the cartridge is inserted; and an upper frame 150 configured to cover and fix the opened top surface of the base frame comprising a guide shaft fixing protrusion fixing one end of each of the guide shafts together with a guide shaft mounting groove of the base frame and a fastening part fastened to the base frame in order to maintain the fixing by the guide shaft fixing protrusion.

The optical module may comprise: a module casing comprising a module lower part and a module upper plate covering and fixing an opened top surface of the module lower part to form an box shaped internal space; a light source disposed within the module casing; a light guide part disposed within the module casing and configured to guide a light from the light source to the barcode of the diagnostic cartridge and to guide a reflected light reflected from the barcode to a light detector; a fluorescence guide part disposed within the module casing comprising a dichroic mirror for guiding a light from the light source to the fluorescence measurement window of the diagnostic cartridge and for guiding a fluorescence emitted from the fluorescence measurement window to a light detector; and a light detector configured to detect the reflected light and the fluorescence; wherein the module lower part on one side thereof has a cylinder barrel into which one of the two guide shafts is put in to guide the optical module in a lengthwise direction, and on the other side thereof has guide protrusions to guide the optical module along the other of the two guide shafts, and on the bottom side thereof has an interrupt protrusion part being inserted into the detection module for a reference position to determine the reference position.

The fluorescence guide part may comprise: a lens for light source for concentrating irradiation light emitted from the light source in a horizontal lateral direction; a dichroic first mirror for changing a path of the irradiation light concentrated by the lens for light source so that the irradiation light travels in a horizontal and front direction, and for transmitting straight the fluorescence emitted from the fluorescence measurement window of the diagnostic cartridge; a second mirror for changing a path of the light whose path has been changed by the dichroic first mirror in a vertical and downward direction to send the light to the fluorescence measurement window of the diagnostic cartridge, and for sending the fluorescence to the first mirror, generated from the fluorescence measurement window of the diagnostic cartridge; a downward lens for concentrating the light whose path has been changed by the second mirror, irradiating the concentrated light to the fluorescence measurement window of the diagnostic cartridge, concentrating the fluorescence emitted from the fluorescence measurement window, and sending the concentrated fluorescence to the second mirror; a detection lens 254 for concentrating the fluorescence whose path has been changed by the second mirror and transmitted straight through the first mirror, and concentrated by the downward lens; and a pin hole 256 for filtering, transmitting and guiding the fluorescence concentrated by the detection lens.

The light source 220 can be one, wherein the light guide part may share a path of light incident on the barcode with the path of the fluorescence guide part, wherein may further comprise a mirror for adjusting the path of light incident on the barcode or a path of light reflected from the barcode so that the reflected light reflected from the barcode is perpendicularly incident on the light detector.

The light detector may comprise a light detector and a fluorescence detector, wherein the light detector can be disposed on the path of the light emitted from the barcode so that the reflected light reflected from the barcode is perpendicularly incident on the light detector.

The light sources can be two, wherein the path of the light incident on the barcode can be different in the light guide part and in the fluorescence guide part, wherein each light emitted by the two light sources can be guided to the detectors along the light guide part and along the fluorescence guide part, respectively, wherein a light source of the light incident on the ambient light guide part can be disposed adjacent to the light detector so that the reflected light reflected from the barcode is perpendicularly incident on the light detector.

The light detector may comprise the light detector and the fluorescence detector, wherein the light source of the light incident on the light guide part and the light detector can be adjacently disposed so that the reflected light reflected from the barcode is perpendicularly incident on the light detector.

The driving module may comprise: a motor configured to provide a rotatory power; a driving shaft 320 connected to the motor in a way to rotate and disposed on at least one lateral side of the optical module, and extending in a front and back direction; and a driving carrier disposed between the optical module and the driving shaft and connected to the optical module and the driving shaft, wherein when the motor rotates and thus the driving shaft is rotated, the driving carrier is displaced in the front and back direction, the two guide shafts are configured to guide the optical module by a cylinder barrel and guide protrusions, and the optical module connected to the driving carrier is guided by the two guide shafts and displaced in the front and back direction.

The cartridge fixing part of the base frame may comprise: a fixing spring configured to lift up and fix a bottom of the diagnostic cartridge; a lifting-up protrusion configured to be matched with and fix a portion of the bottom groove of the diagnostic cartridge; and a side fitting frame 134 configured to fix the diagnostic cartridge by inserting a side of the diagnostic cartridge therein.

The reference position detection module may comprise: a module main board fixed in a direction opposite to the entrance part of the base frame; and an interrupt sensor part disposed in a direction opposite to the entrance part on the module main board, wherein the interrupt protrusion part can be inserted into the interrupt sensor part, wherein a position of the optical module can be displaced between the reference position where the interrupt protrusion part is inserted into the interrupt sensor part and a position of the entrance part.

The reference position detection module may further comprise a cartridge sensing switch disposed under the module main board, wherein the diagnostic cartridge can be configured to press the cartridge sensing switch when the diagnostic cartridge is inserted into the base frame through the entrance part and an end of the diagnostic cartridge reaches a specific position, wherein the cartridge sensing switch may generate an insertion signal for the diagnostic cartridge when the diagnostic cartridge presses the cartridge sensing switch.

The diagnostic cartridge may comprise the fluorescence measurement window extended in a lateral flow direction of a sample and having the lateral flow of the sample exposed upward, and a barcode for identifying a type of sample, wherein the optical module can be configured to: identify the type of sample by reading the barcode by moving to the barcode area, move to an end point of the fluorescence measurement window after detecting lateral flow start time of the sample at a start point of the fluorescence measurement window, and calculate a flow rate of the lateral flow by detecting lateral flow end time of the sample at the end point of the fluorescence measurement window.

The diagnostic cartridge may comprise the fluorescence measurement window extended in a lateral flow direction of a sample and having the lateral flow of the sample exposed upward, and a barcode, wherein the optical module can be configured to: identify the type of sample by reading the barcode by moving for the barcode light source to irradiate a light to the barcode; move to an end point of the fluorescence measurement window after detecting lateral flow start time of the sample at a start point of the fluorescence measurement window, and calculate a flow rate of the lateral flow by detecting lateral flow end time of the sample at the end point of the fluorescence measurement window.

The immunodiagnostic fluorescence reader according to the present invention can prevent an error by identifying barcode although different types of diagnostic cartridges are inserted using barcode information formed on the same surface as the fluorescence measurement window of the diagnostic cartridge, can automatically calculate a reaction time by detecting a sample flowing into a membrane when the sample is loaded, and can simultaneously analyze the type of diagnosis marker or sample by detecting fluorescent information measured through the fluorescence measurement window.

### [Description of Drawings]

FIG. 1 is a diagram illustrating an integrated immunodiagnostic fluorescence reader having multiple diagnosis function according to the present disclosure.
FIG. 2 is a diagram illustrating an optical module of the integrated immunodiagnostic fluorescence reader having multiple diagnosis function according to the present disclosure.
FIG. 3 is a diagram illustrating parts of the optical module in detail.
FIG. 4 is a diagram illustrating a diagnostic cartridge of the integrated immunodiagnostic fluorescence reader having multiple diagnosis function according to the present disclosure.
FIG. 5 is a diagram illustrating the optical module and a driving module.
FIG. 6 is a diagram illustrating a position detection module of the integrated immunodiagnostic fluorescence reader having multiple diagnosis function according to the present disclosure.
FIG. 7 is a diagram illustrating one cross section of the integrated immunodiagnostic fluorescence reader having multiple diagnosis function according to the present disclosure on which the optical module and the driving module are mounted.
FIG. 8 is a plan view illustrating a base frame of the integrated immunodiagnostic fluorescence reader having multiple diagnosis function according to the present disclosure.
FIG. 9 is a diagram illustrating a structure in which a guide shaft is fixed by a base frame and an upper frame.
FIG. 10 is a diagram illustrating part of a cross section of the integrated fluorescence reader for multi-diagnosis on which the diagnostic cartridge is mounted.

### [Detailed Description of Embodiments]

Hereinafter, preferred embodiments according to the present disclosure are described with reference to the accompanying drawings.

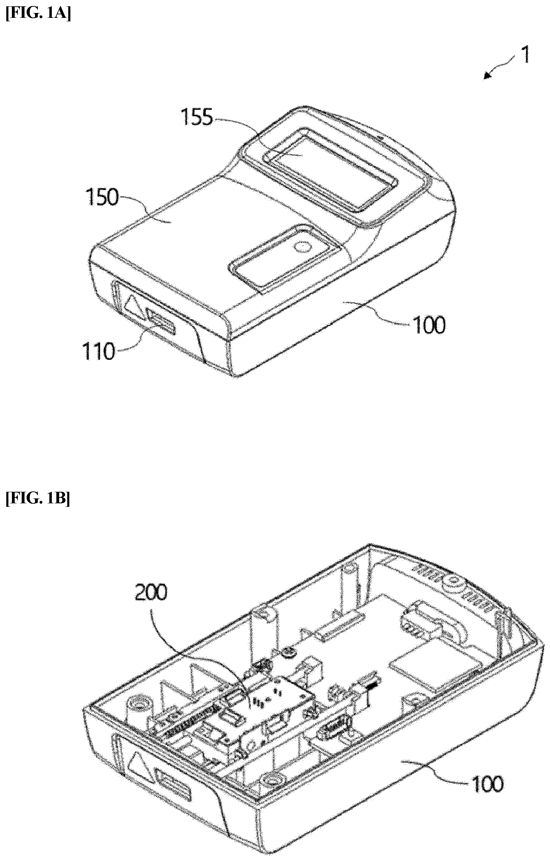

FIG. 1 is a diagram illustrating an integrated immunodiagnostic fluorescence reader having multiple diagnosis function1 having a multi-diagnosis function according to the present disclosure. In FIG. 1, an X-axis direction is denoted as a "front and back direction", a Y-axis direction is denoted as a "lateral direction", and a Z-axis direction is denoted as a "height direction." When "the front and back direction", "the "lateral direction", or "the height direction" is mentioned, the direction is determined based on a direction indicated in FIG. 1.

FIG. 1A illustrates a state in which an upper frame 150 of the integrated immunodiagnostic fluorescence reader having multiple diagnosis function1 having a multi-diagnosis function is covered. FIG. 1B illustrates a base frame 100 and an internal shape thereof in the state in which the upper frame 150 is not covered.

The integrated immunodiagnostic fluorescence reader having multiple diagnosis function1 having a multi-diagnosis function according to an embodiment of the present disclosure is configured to comprise the base frame 100, the upper frame 150, an optical module 200, a driving module 300, and a reference position detection module 400.

In one embodiment of the present disclosure, the base frame 100 constitutes a lower external appearance of the integrated immunodiagnostic fluorescence reader having multiple diagnosis function1 having a multi-diagnosis function according to the present disclosure. The base frame 100 is configured so that the optical module 200, the driving module 300, the position detection module 400, and various elements are properly fixed or mounted. In one embodiment of the present disclosure, the upper frame 150 constitutes an external appearance of the integrated immunodiagnostic fluorescence reader having multiple diagnosis function1 having a multi-diagnosis function in a form engaged with the base frame 100 to cover the base frame 100, and comprises a display part 155. The display part 155 is preferably integrated and configured with the upper frame 150, but may be configured as a separate frame and combined with the upper frame. In one embodiment of the present disclosure, the display part 155 displays an operating state of the integrated immunodiagnostic fluorescence reader having multiple diagnosis function1 having a multi-diagnosis function according to the present disclosure and results according to an operation thereof through a display window.

In one embodiment of the present disclosure, a given entrance part 110 is formed at one side of the base frame 100. The base frame 100 has a given insertion space 120 opened to the outside through the entrance part 110, and is configured to enable a diagnostic cartridge to be inserted into the insertion space 120 through the entrance part 110.

In one embodiment of the present disclosure, the base frame 100 may comprise a cartridge fixing part 130 as fixing means for preventing a diagnostic cartridge from being unnecessarily shaken or detached when the diagnostic cartridge is inserted into the insertion space 120. Furthermore, the base frame 100 may be provided with a given connector so that a given electric device, a power supply, etc. are connected thereto, but the present disclosure is not limited thereto.

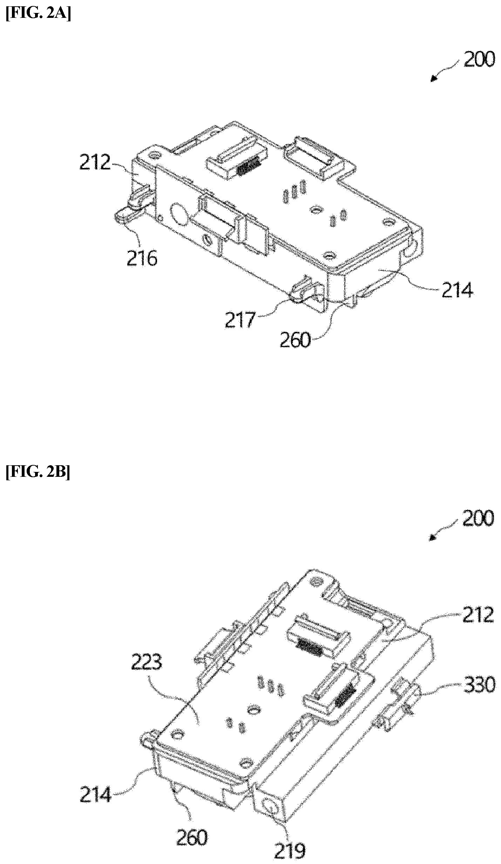

FIG. 2 is a diagram illustrating the optical module 200 of the integrated immunodiagnostic fluorescence reader having multiple diagnosis function1 having a multi-diagnosis function according to the present disclosure. FIG. 2A is a diagram viewed from a direction in which one side of the optical module 200, that is, a first guide protrusion 216 and a second guide protrusion 217, are present in the state in which an optical module upper plate 212 has been covered. FIG. 2B is a diagram viewed from a direction in which another aspect of the optical module, that is, a cylinder barrel 219, is present and a driving carrier 330 has been attached.

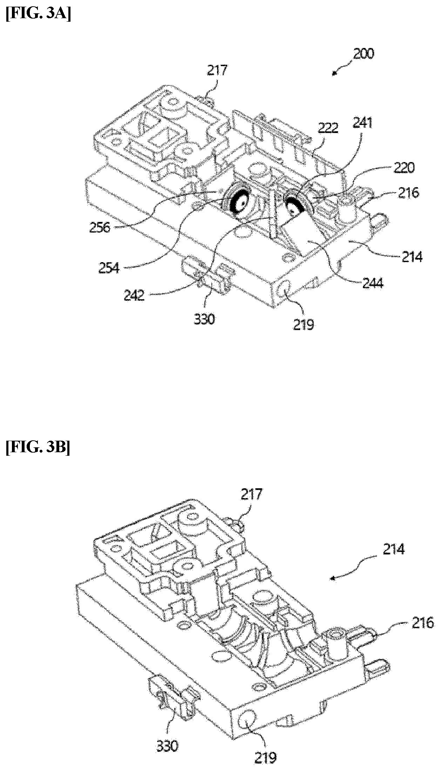

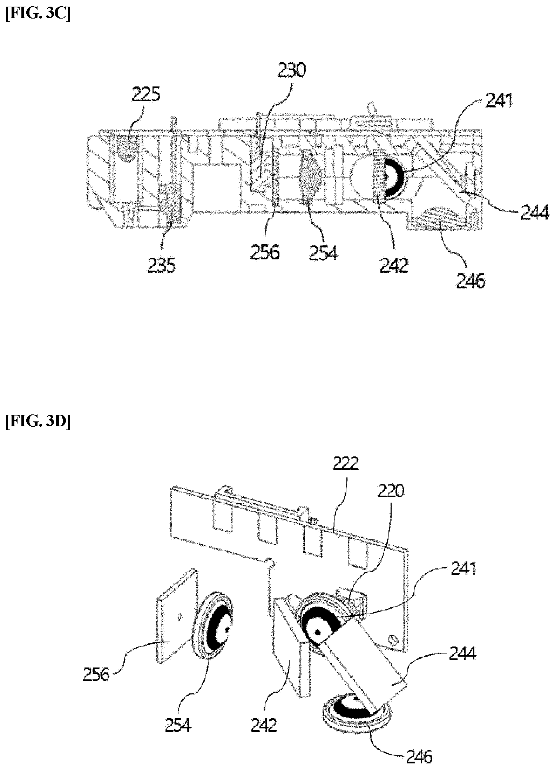

FIG. 3 is a diagram illustrating parts of the optical module 200 in detail. FIG. 3A is a diagram illustrating an optical module lower part 214, a light source 220, a fluorescence guide part, etc. in the state in which the optical module upper plate 212 has not been covered. FIG. 3B is a diagram illustrating only the optical module lower part 214 in the state in which the light source 220, an ambient light guide part, etc. have not been disposed. FIG. 3C is a cross-sectional view illustrating optical parts, such as the fluorescence guide part, the ambient light guide part, and a light detector 230. FIG. 3D is a perspective view illustrating some of optical parts, such as the light source 220, the fluorescence guide part, etc.

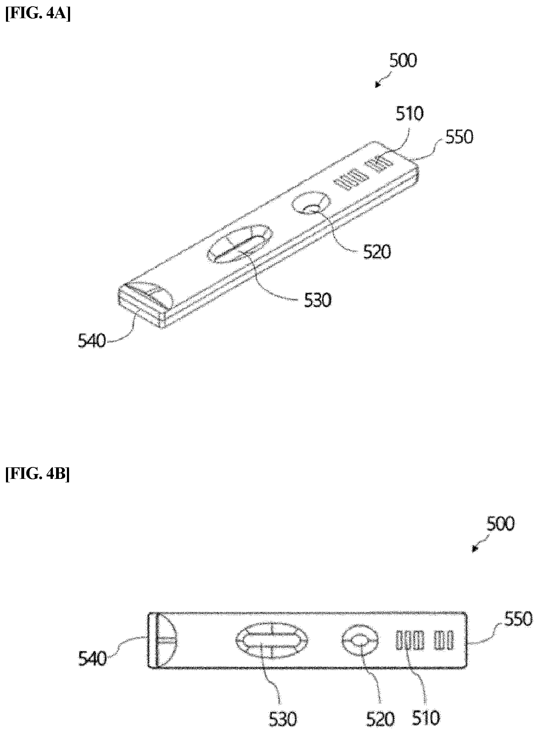

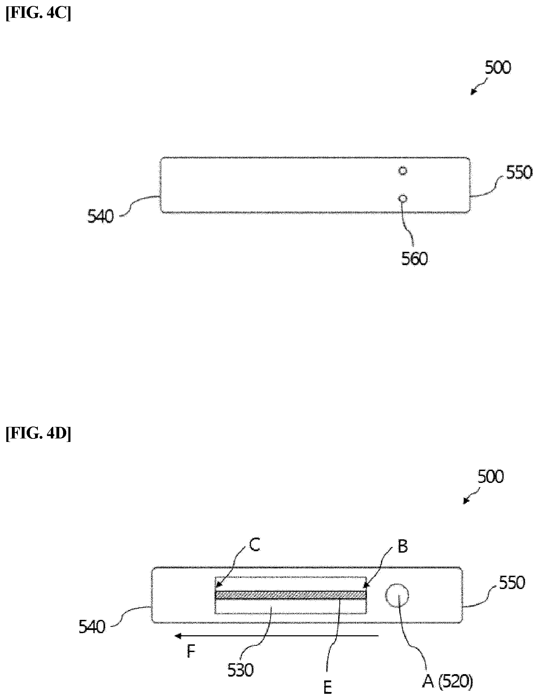

FIG. 4 is a diagram illustrating a diagnostic cartridge 500 of the integrated immunodiagnostic fluorescence reader having multiple diagnosis function1 having a multi-diagnosis function according to the present disclosure. FIG. 4A is a perspective view of the diagnostic cartridge 500. FIG. 4B is a top plan view of the diagnostic cartridge. FIG. 4C is a plan view of the bottom of the diagnostic cartridge. FIG. 4D is a diagram illustrating a process of a sample being deployed in the diagnostic cartridge.

Referring to FIG. 4, the top surface of the diagnostic cartridge 500 comprises the portion of barcode 510 differently assigned for each diagnosis marker and a fluorescence measurement window 530 capable of measuring fluorescence emission light from a fluorescent material changed by an input sample. A sample inlet 520 to which a sample is input is provided between the barcode 510 and the fluorescence measurement window 530. A bottom groove 560 is provided at the bottom of the diagnostic cartridge 500. Among ends of the diagnostic cartridge 500, a side opposite to the barcode 510, that is, a side close to the fluorescence measurement window 530, is called a first end 540, and an end on the side where the barcode 510 is located is called a second end 550.

In one embodiment of the present disclosure, the optical module 200 is provided to radiate a given light to the diagnostic cartridge 500 inserted into the base frame 100 and to detect fluorescence emission light and barcode-ambient light of the incident light emitted after the incident light is radiated to the fluorescence measurement window 530 and barcode 510 of a sample.

In one embodiment of the present disclosure, the barcode reading system may use commercial one-dimensional or two-dimensional barcode. However, the one-dimensional barcode has a disadvantage in that a price rises due to increased complexity of a device in reading a close cartridge because a corresponding structure is large and a focal distance is long. The two-dimensional barcode may be properly selected with complexity because it comprises a large amount of information compared to the one-dimensional barcode, and requires a high-performance processor and an image processing technique for two-dimensional decoding. For this reason, a cost may rise because a CMOS or CCD camera module is used. Accordingly, in one embodiment of the present disclosure, multiple diagnosis markers can be identified using unique one-dimensional barcode specialized for cartridge identification without using commercial barcode. To this end, a method of identifying a difference between signals of the unique one-dimensional barcode using a light source and a detection unit and classifying the signals in a binary number unit may be used. In one embodiment of the present disclosure, the unique one-dimensional barcode may have a shape in which a plurality of solid-line parts are isolated at predetermined intervals, and may preferably comprise five or more solid-line parts, but the present disclosure is not limited thereto.

In one embodiment of the present disclosure, the optical module 200 may be configured to comprise the module upper plate 212 and the module lower part 214 forming a module casing, the light sources 220 and 225, the fluorescence guide part, the ambient light guide part, the light detectors 230 and 235, an interrupt protrusion part 260, and various PCBs and connectors.

The module casing is configured as a casing having a given box form, has a space formed therein, and is configured to have the light source 220, the fluorescence guide part, the ambient light guide part, and the detectors 230 and 235 embedded and installed therein. In one embodiment of the present disclosure, the module casing has a stereoscopic shape having a generally rectangular box form, and preferably has a construction coupled by the module lower part 214 and the module upper plate 212 that covers and fixes an opened top of the module lower part 214, but the present disclosure is not essentially limited thereto.

Various boards and connectors may be provided on the outside of the module casing. In one embodiment of the present disclosure, the connectors are provided so that the light sources 220 and 225, the detectors 230 and 235, etc. within the module casing exchange electrical signals with an external electrical device, and may be connected to given electrical terminals.

The module casing, in particular, the module lower part 214 may be equipped with given guide parts disposed on the outside of a side thereof on both sides. In one embodiment of the present disclosure, the guide part is a member for stably guiding a displacement of the module casing when the module casing is displaced in the front and back direction by the driving module 300 to be described later.

The guide part comprises a cylinder barrel 219 on one side thereof and guide protrusions 216 and 217 on the other side thereof so that two guide shafts 352 and 354 to be described later are guided. That is, the cylinder barrel 219 that guides the first guide shaft 352 to be inserted in a lengthwise direction thereof is provided on one side of the module lower part 214. The first guide protrusion 216 and the second guide protrusion 217 guided along the second guide shaft 354 are provided on the other side of the module lower part 214. However, the first guide shaft 352 does not need to be essentially guided only in the form of a cylinder barrel, and may be guided in the form of a guide protrusion like the second guide shaft 354.

In one embodiment of the present disclosure, the first guide protrusion 216 is preferably configured to comprise two upper and lower protrusions at one end on one side where the second guide shaft 354 is located. The second guide protrusion 217 is preferably configured as one protrusion at the other end on one side where the second guide shaft 354 is located, but the present disclosure is not essentially limited thereto. An upper and down width between the upper protrusion and lower protrusion of the first guide protrusion 216 may correspond to the diameter of the second guide shaft 354.

In one embodiment of the present disclosure, the light source 220 and/or the barcode light source 225 are disposed within the module lower part 214 of the module casing, and may generate a light having a given wavelength by being applied with given power. For example, the light source 220 and the barcode light source 225 may be configured as light-emitting diodes (LED) for generating light having a specific wavelength band or may be configured as given laser light sources for generating straight-line light, but the present disclosure is not essentially limited thereto.

In an embodiment of the present disclosure illustrated in the drawings, a case where the light source 220 and the barcode light source 225 are separately provided, that is, two light sources are present, is described as an example, but the present disclosure is not essentially limited thereto. That is, the present disclosure may be configured so that the light source comprises the one light source 220 to also play a role as the barcode light source. In one embodiment of the present disclosure, if the two light sources are used, the light source board 222 for fluorescence for controlling an operation of the light source 220 used as a light source for fluorescence and the light source board 223 for barcode for controlling an operation of the barcode light source 225 may be provided.

In one embodiment of the present disclosure, the fluorescence guide part is disposed within the module casing to guide a light generated by the light source 220 to the fluorescence measurement window 530 of the diagnostic cartridge 500, and to guide fluorescence emitted from the fluorescence measurement window to the fluorescence detector 230. Accordingly, the light generated by the light source 220 is guided by the fluorescence guide part, radiated to the fluorescence measurement window 530 of the diagnostic cartridge 500, and incident on a given fluorescent material comprised in the sample loaded onto the diagnostic cartridge 500, so that fluorescence emission light is generated by the fluorescent material.

The ambient light guide part is disposed within the module casing to guide a light generated by the barcode light source 225 to the barcode 510 of the diagnostic cartridge 500, and to guide ambient light, such as reflected light, diffused light, and scattered light reflected from the barcode 510, to the ambient light detector 235. In an embodiment of the present disclosure illustrated in the drawings, the ambient light guide part has been illustrated as being comprised separately from the fluorescence guide part. However, if the number of light sources is one, the fluorescence guide part may be configured to partially play a role as the ambient light guide part.

The fluorescence detector 230 and the ambient light detector 235 are configured to obtain fluorescence emission light and ambient light generated from the diagnostic cartridge 500. In one embodiment, photodiodes are used as the fluorescence detector 230 and the ambient light detector 235. Fluorescence emission light and ambient reflected light are converted into an electrical signal through the photodiodes. In another embodiment, the fluorescence detector 230 and the ambient light detector 235 may be composed of different types of photo detectors.

In an embodiment of the present disclosure illustrated in the drawings, a case where the fluorescence detector 230 and the ambient light detector 235 are separately provided, that is, two light detectors are present, is described as an example, but the present disclosure is not essentially limited thereto. That is, the present disclosure may be configured so that the fluorescence detector comprises only the one light detector 230 to also play a role as the ambient light detector.

The interrupt protrusion part 260 may be provided to protrude at one end at the bottom of the module lower part 214. The interrupt protrusion part 260 is configured as a protrusion part that protrudes with a given width and length. The interrupt protrusion part 260 controls an operation of an interrupt sensor part 420 to be described later, and a detailed configuration and operation of the interrupt sensor part 420 are described later.

Hereinafter, if the optical module 200 according to an embodiment of the present disclosure comprises the two light sources 220 and 225 and the two light detectors 230 and 235 as illustrated in the drawings, the fluorescence guide part and the ambient light guide part are described in detail.

In one embodiment of the present disclosure, the fluorescence guide part comprises a lens for light source 241, a first mirror 242, a second mirror 244, a downward lens 246, a detection lens 254, and a pin hole 256. The lens for light source 241 concentrates irradiation light emitted in a horizontal and lateral direction from the light source 220, and sends the light to the first mirror 242. The first mirror 242 changes the path of the irradiation light concentrated by the lens for light source 241 so that the irradiation light travels in a horizontal and front direction, and reflects the light. That is, the first mirror 242 is inclined laterally to the path of light laterally emitted by the light source 220 so that the path of the light is changed to the front direction. Specifically, assuming that light radiated by the light source 220 is incident on an XY plane in a Y-axis direction, the first mirror 242 may have an orientation angle that is perpendicular to the XY plane and inclined by 45° with respect to the Y-axis. The first mirror 242 is a dichroic mirror and has both a filter function and a reflection mirror function. That is, the first mirror 242 is an element that reflects a wavelength band of irradiation light and transmits a wavelength band of fluorescence emission light.

The second mirror 244 changes the path of the light whose path has been changed by the dichroic first mirror 242 in a perpendicular and downward direction, and sends the light toward the downward lens 246 and the fluorescence measurement window 530 of the diagnostic cartridge 500. The second mirror 244 is a total reflection mirror, and is downward inclined to downward change the path of light radiated in the front direction. Specifically, assuming that light reflected by the first mirror 242 is incident on the XY plane in the X-axis direction, the second mirror 244 may have an orientation angle that is perpendicular to the XZ plane and inclined by 45° with respect to the X axis.

The downward lens 246 concentrates the light whose path has been changed by the second mirror 244, and radiates the concentrated light to the fluorescence measurement window 530 of the diagnostic cartridge 500. Furthermore, the downward lens 246 concentrates fluorescence emission light generated due to the light radiated to the fluorescence measurement window 530, and sends the concentrated light to the second mirror 244 again. The fluorescence emission light concentrated by downward lens 246 is reflected by the second mirror 244, so that the fluorescence emission light having a direction changed is sent to the first mirror 242 again. The first mirror is a dichroic mirror, and thus directs the fluorescence emission light toward the detection lens 254 by transmitting the fluorescence emission light without reflecting the fluorescence emission light. That is, the fluorescence emission light transmits straight through the first mirror 242. The detection lens 254 concentrates the fluorescence emission light passing through the first mirror and directs the concentrated light toward the pin hole 256.

In one embodiment of the present disclosure, the pin hole 256 filters, transmits, and guides the fluorescence emission light concentrated by the detection lens 254. The fluorescence emission light filtered by the pin hole 256 is received by the fluorescence detector 230. The pin hole 256 may be configured as a given hole that enables only fluorescence emission light within a specific path to be incident on the fluorescence detector 230. Each of the lenses 241, 246, and 254 may be configured as a given device that properly modulates and converts light or fluorescence emission light. The fluorescence detector 230 is equipped with a converter. Accordingly, light received by the fluorescence detector 230 is converted into a voltage signal through the converter.

In the configuration of the embodiment comprising the two light sources 220 and 225 and the two light detectors 230 and 235, the ambient light guide part does not comprise a separate lens, a separate mirror, etc. That is, the ambient light guide part directly guides, to the barcode 510 of the diagnostic cartridge 500, light generated by the barcode light source 225, and guides ambient light reflected from the barcode 510 to be perpendicularly incident on the ambient light detector 235.

Although not illustrated in the drawing in one embodiment of the present disclosure, in another embodiment of the present disclosure comprising only the one light source 220, the ambient light guide part is also differently configured. That is, the ambient light guide part shares the path of light incident on the barcode with the fluorescence guide part, so that light radiated by the light source 220 is incident on the barcode 510 of the diagnostic cartridge 500 via the lens for light source 241, the first mirror 242, the second mirror 244, and the downward lens 246. However, ambient light reflected from the barcode 510 does not pass through the first mirror 242, that is, the dichroic mirror of the fluorescence guide part. For this reason, a separate lower pin hole (not illustrated) is provided to adjust the path of ambient light reflected from the barcode so that the light is perpendicularly incident on the light detector 230.

In this case, that is, if one light source is present, one light detector may be configured to perform both fluorescence detection and ambient light detection functions. Two detectors, that is, the fluorescence detector 230 and the ambient light detector 235, may be configured. Regardless of the number of detectors, a light detector that receives light around the barcode may comprise a lower pin hole (not illustrated) thereunder, and may filter, transmit, and guide the received light. Accordingly, if only one light source 220 is provided, the ambient light guide part comprises optical elements such as the lower pin hole.

Although not illustrated, in one embodiment of the present disclosure, another embodiment of the present disclosure comprising the two light sources 220 and 225 and one light detector 230 is possible. In this case, the ambient light guide part is differently configured. If two light sources, that is, the light source 220 for fluorescence radiation and the separate barcode light source 225 are provided, the ambient light guide part does not need to share the path of light incident on the barcode with the fluorescence guide part. Accordingly, similar to the case comprising the two light sources 220 and 225 and the two light detectors 230 and 235, light generated by the barcode light source 225 is directly guided to the barcode 510 of the diagnostic cartridge 500, and ambient light reflected from the barcode 510 is guided to be perpendicularly incident on the fluorescence detector 230.

However, since the ambient light detector is configured to also perform the fluorescence detection function, the lower pin hole needs to be provided to filter, transmit, and guide ambient light. For example, if the two light sources 220 and 225 and the one light detector 230 are provided, the ambient light guide part needs to comprise the lower pin hole.

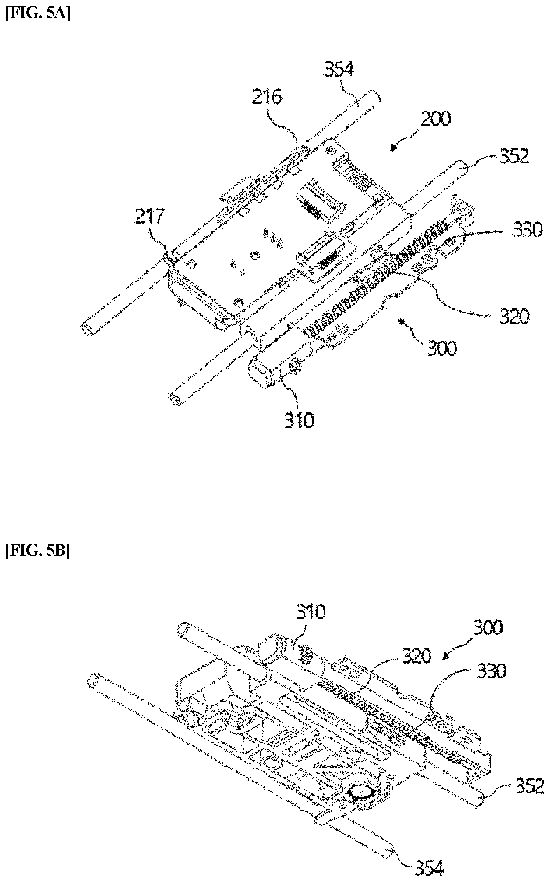

FIG. 5 is a diagram illustrating the optical module 200 and driving module 300 of the integrated immunodiagnostic fluorescence reader having multiple diagnosis function1 having a multi-diagnosis function according to the present disclosure. FIG. 5A is a perspective view of a structure in which the optical module 200 and the driving module 300 are coupled, which is viewed from the top. FIG. 5B is a perspective view of the structure in which the optical module 200 and the driving module 300 are coupled, which is viewed from the bottom.

In one embodiment of the present disclosure, the driving module 300 consists of a device that provides or delivers motive power to displace the optical module 200 or a device that guides a displacement of the optical module 200. Specifically, the driving module 300 may be configured to comprise a motor 310, a driving shaft 320, a driving carrier 330, and guide shafts 352 and 354.

In one embodiment of the present disclosure, the motor 310 is provided to generate a rotatory power by receiving given electric power. The motor 310 may be disposed at an edge portion on one side within the internal space of the casing.

The driving shaft 320 is configured as a given shaft that is coupled to the motor 310 and rotated. The driving shaft 320 is disposed to elongate front and back in parallel to the optical module 200 on at least one outside of the optical module 200. In one embodiment of the present disclosure, the driving carrier 330 is a member coupled to one side of the optical module 200, and is a member that couples the optical module 200 and the driving shaft 320 and that achieves a front and back displacement of the driving module 300 using a rotatory power delivered to the driving shaft 320. One side of the driving carrier 330 is connected to the optical module 200. A given elastic part (no reference numeral) may be provided between one side and the other side of the driving carrier 330.

In one embodiment of the present disclosure, one side of the driving carrier 330 is configured to be fixed to the module lower part 214 of the optical module casing, and is preferably fixed to one side where the cylinder barrel 219 is located among sides of the module lower part 214. Furthermore, the driving carrier 330 and the optical module 200 may be coupled and integrated. When an external force is applied to the driving carrier 330, the carrier 330 and the optical module 200 may be displaced at the same time.

The guide shafts 352 and 354 may be configured as given beams that are disposed on both sides on the outside of the optical module 200, respectively, and that are elongated front and back. For example, the first guide shaft 352 located between the driving shaft 320 and the optical module 200 and the second guide shaft 354 located on a side opposite to the side where the first guide shaft 352 is located may be provided. The two guide shafts 352 and 354 each have one end fixed to the guide shaft fitting groove 145 of the base frame 100 to be described later and the other end cradled in the guide shaft mounting groove 140 of the base frame, so the two guide shafts are disposed in parallel to the lengthwise direction of the diagnostic cartridge 500 inserted into the insertion space 120.

As described above, the guide parts are provided on both sides of the module lower part 214 of the casing of the optical module 200. The cylinder barrel 219 is provided on one side of the optical module 200, that is, the side to which the driving carrier 330 is coupled and fixed, enabling the first guide shaft 352 to be inserted and guided in the lengthwise direction thereof. The first guide protrusion 216 and the second guide protrusion 217 are provided on the other side of the optical module 200 and guided along the second guide shaft 354.

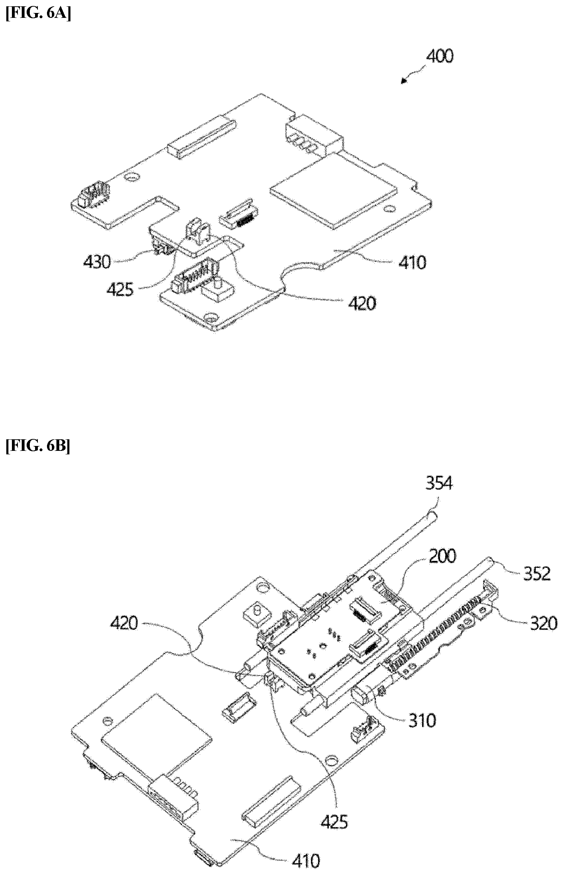

FIG. 6 is a diagram illustrating the reference position detection module of the integrated immunodiagnostic fluorescence reader having multiple diagnosis function1 having a multi-diagnosis function according to the present disclosure. FIG. 6A is a perspective view illustrating only the reference position detection module. FIG. 6B is a perspective view illustrates a shape in which the reference position detection module 400 and the optical module 200 are connected.

The reference position detection module 400 is configured to comprise a sensor that detects the insertion of the diagnostic cartridge 500 and a position of the optical module 200. The reference position detection module 400 is configured to comprise a module main board 410, an interrupt sensor part 420, and a cartridge sensing switch 430.

The module main board 410 may be configured as a given PCB that is located within the base frame 100 and fixedly coupled thereto. Preferably, the module main board 410 may be disposed at a position in a direction opposite to the entrance part 110 on the side at the front of the base frame 100. The interrupt sensor part 420 may be disposed over the module main board 410, and the cartridge sensing switch 430 may be disposed under the module main board 410. Furthermore, a given connector may be provided.

In one embodiment of the present disclosure, the interrupt sensor part 420 has a given insertion groove formed at the front thereof, and is composed of an insertion detection sensor 425 located within the groove. That is, the interrupt sensor part 420 has the insertion groove formed at the front thereof, and part of the front is depressed. The interrupt sensor part 420 may be composed of a given insertion detection sensor 425 from which a signal is generated or by which a signal is blocked when a given device or obstacle is inserted into the insertion groove.

The interrupt protrusion part 260 provided in the optical module 200 may be inserted into the insertion groove formed in the interrupt sensor part 420. A distance between the interrupt sensor part 420 and the interrupt protrusion part 260 varies as the optical module 200 is displaced. When the optical module 200 becomes adjacent to the module main board 410, the interrupt protrusion part 260 is inserted into the interrupt sensor part 420. The insertion detection sensor 425 of the interrupt sensor part 420 may generate a given signal or stop a signal when the interrupt protrusion part 260 is inserted into the interrupt sensor part 420. Specifically, the interrupt sensor part 420 according to the present application may detect the out-of phase of the optical module 200. The out-of phase means that the optical module 200 is not accurately moved along a rail by an instructed step. In order to solve the out-of phase, a position that becomes a reference is determined, and the optical module 200 is moved from the position by an instructed step. The interrupt sensor part 420 enables the reference position to be recognized. That is, when the interrupt protrusion part 260 provided in the optical module 200 is inserted into the insertion groove formed in the interrupt sensor part 420, the reference position is recognized. The optical module first moves to the reference position before moving from one point to another point, and then moves from the reference position by an instructed step.

In one embodiment of the present disclosure, the cartridge sensing switch 430 is configured as a given switch that senses whether the diagnostic cartridge 500 has been inserted into an accurate depth within the base frame 100. For example, when the diagnostic cartridge 500 is inserted up to a given depth and reaches a position where test can be properly performed, the sensing switch 430 may be pressed by the diagnostic cartridge 500 to generate a signal, which is described in detail later.

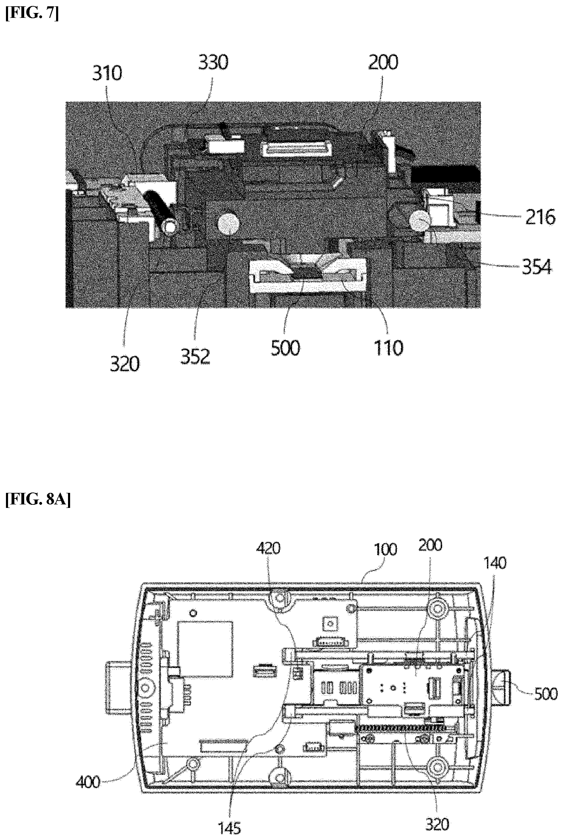

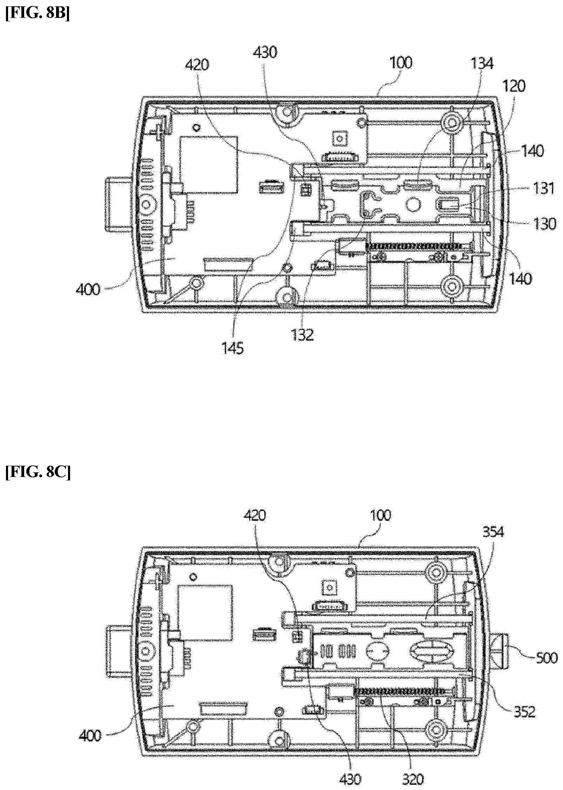

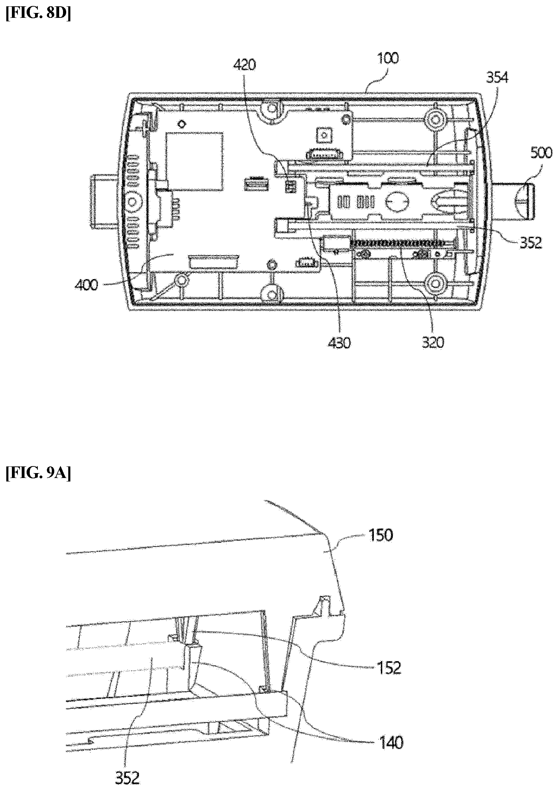

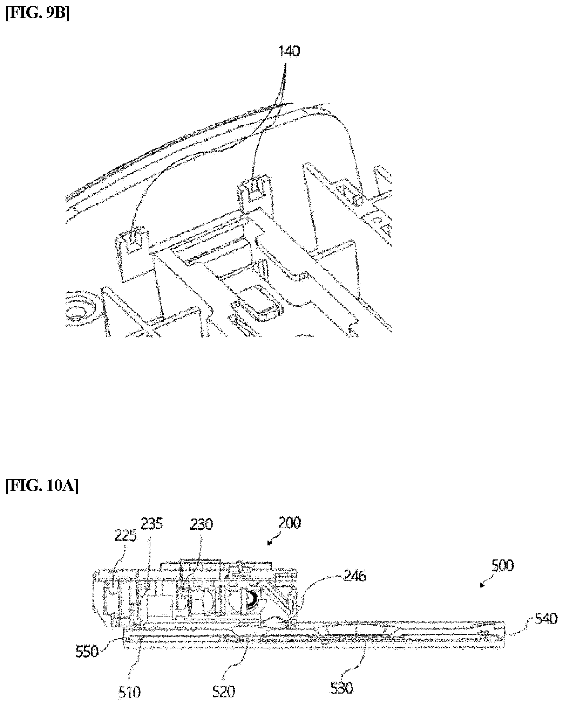

FIG. 7 is a diagram illustrating one cross section of the integrated immunodiagnostic fluorescence reader having multiple diagnosis function1 having a multi-diagnosis function according to the present disclosure, on which the optical module 200 and the driving module 300 are mounted. FIG. 8 is a plan view illustrating the base frame 100 of the integrated immunodiagnostic fluorescence reader having multiple diagnosis function1 having a multi-diagnosis function according to the present disclosure. FIG. 8A is a plan view of a state in which the optical module 200 is mounted on and the diagnostic cartridge 500 is inserted into the base frame 100. FIG. 8B is a plan view of a state in which the optical module 200 and the diagnostic cartridge 500 are not mounted on the base frame 100. FIG. 8C is a plan view illustrating a state in which the diagnostic cartridge 500 has been inserted to the end of the base frame in the state in which the optical module has not been mounted on the base frame. FIG. 8D is a plan view illustrating a state right before the diagnostic cartridge 500 is inserted to the end of the base frame. FIG. 9 is a diagram illustrating a structure in which the guide shafts 352 and 354 are fixed by the base frame 100 and the upper frame 150.

In one embodiment of the present disclosure, the base frame 100 has an opened top surface, and comprises the entrance part 110 into which the diagnostic cartridge 500 is inserted on a side at the front thereof and the insertion space 120 opened to the outside through the entrance part 110 on the side at the front. Furthermore, a space for the movement of the optical module is located on an upper part of the insertion space 120, so that the optical module is moved along the two guide shafts 352 and 354 fixed to both sides in parallel to the lengthwise direction of the diagnostic cartridge 500 inserted into the insertion space 120.

In one embodiment of the present disclosure, a space for driving module mounting is located on one side of the space for the movement of the optical module. A space for reference position detection module mounting that detects the reference position of the optical module 200 is also provided within the base frame 100.

The upper frame 150 covers the opened top of the base frame 100 and fixes the top thereto. The guide shaft fixing protrusion 152 fixes the end of each of the guide shafts 352 and 354 along with the guide shaft mounting groove 140 of the base frame 100. That is, one end of each of the guide shafts 352 and 354 is fit into and fixed by the guide shaft fitting groove 145 of the base frame. The other ends of the guide shafts 352 and 354 are cradled in the guide shaft mounting groove 140 of the base frame 100 and simultaneously pressed and fixed by the guide shaft fixing protrusion 152. The upper frame 150 comprises a fastening part fastened to the base frame 100 in order to maintain the fixing of the guide shaft fixing protrusion 152.

The cartridge fixing part 130 is provided in the insertion space 120 under the space for the movement of the optical module within the base frame 100. When the diagnostic cartridge 500 is inserted into the insertion space 120, the cartridge fixing part 130 fixes the position of the diagnostic cartridge 500 by applying pressure to at least part of the diagnostic cartridge.

Referring to FIG. 8B, the cartridge fixing part 130 of the base frame 100 comprises a fixing spring 131, a lifting-up protrusion 132, and a side fitting frame 134. The fixing spring 131 is a member having an elastic force, and lifts up and fixes the bottom of the diagnostic cartridge 500. The lifting-up protrusion 132 is matched with a portion of the bottom groove 560 of the diagnostic cartridge 500, and helps the fixing. The side of the diagnostic cartridge 500 is inserted into the side fitting frame 134, and thus the diagnostic cartridge 500 is fixed by the side fitting frame 134.

Referring to FIGS. 8C and 8D, when the diagnostic cartridge 500 is inserted up to a proper depth within the insertion space 120 through the entrance part 110 on the side at the front thereof and reaches a regular position, the second end 550 of the diagnostic cartridge 500 generates an insertion signal for the diagnostic cartridge 500 while pressing the cartridge sensing switch 430 by pushing the cartridge sensing switch 430. In this case, the cartridge sensing switch 430 may be configured to comprise a button having a given shape so that the cartridge sensing switch 430 has a construction to be pushed and pressed by the second end 550 of the diagnostic cartridge 500.

As described above, the interrupt sensor part 420 is provided over the module main board 410, the cartridge sensing switch 430 is provided under the module main board 410, and the interrupt sensor part 420 and the cartridge sensing switch 430 are integrated with the module main board 410. Accordingly, a reduction in position accuracy attributable to an assembly error can be prevented. That is, for example, if the interrupt sensor part 420 and the cartridge sensing switch 430 are independently moved or independently assembled, an error occurrence possibility may rise. In contrast, according to the present disclosure, an error occurrence possibility can be reduced and accurate test can be performed because the interrupt sensor part 420 detecting a position of the optical module 200 and the cartridge sensing switch 430 detecting a position of the diagnostic cartridge 500 are together provided in the one module main board 410.

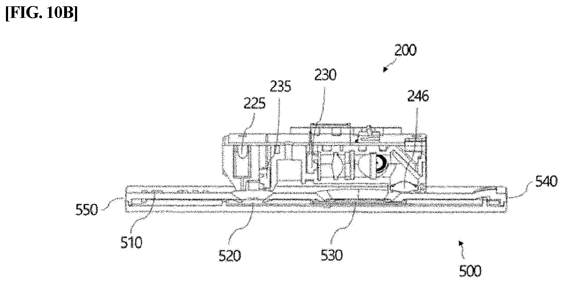

Hereinafter, an operation of the integrated immunodiagnostic fluorescence reader having multiple diagnosis function1 having a multi-diagnosis function according to the present disclosure is described. FIG. 10 is a diagram illustrating part of a cross section of the integrated fluorescence reader for multi-diagnosis on which the diagnostic cartridge 500 is mounted. FIG. 10A is a cross-sectional view if the optical module 200 reads the barcode 510 of the diagnostic cartridge 500. FIG. 10B is a cross-sectional view if the optical module 200 reads the fluorescence measurement window 530 of the diagnostic cartridge 500.

When the diagnostic cartridge 500 is inserted, first, the optical module 200 moves to a position where the barcode 510 of the diagnostic cartridge 500 can be properly read. That is, at a position where light emitted from the barcode light source 225 can be properly radiated to the barcode 510 of the diagnostic cartridge 500 through the ambient light guide part, the barcode detector 235 may receive light reflected from the barcode 510 and identify a diagnosis marker, the type of sample, etc. based on assigned barcode information.

After the barcode 510 of the diagnostic cartridge 500 is read, the optical module 200 moves to a position where the fluorescence measurement window 530 of the diagnostic cartridge 500 can be properly read. That is, the position is a location where light emitted by the light source 220 of the optical module can be properly radiated to the fluorescence measurement window 530 of the diagnostic cartridge 500 through the fluorescence guide part. The light is finally radiated to the sample in the portion of the fluorescence measurement window 530 of the diagnostic cartridge 500 located under the fluorescence measurement window 530 through the downward lens 246.

The sample of the diagnostic cartridge 500 comprises a given fluorescent material and thus generates fluorescence emission light when the light is incident on the sample. The fluorescence emission light is upward emitted. As described above, the fluorescence emission light passes through the downward lens 246 and is received by the fluorescence detector 230 via the second mirror 244, the first mirror 242, the detection lens 254, and the pin hole 256.

As illustrated in FIG. 4D, after lateral flow start time of the sample is detected at the start point B of the fluorescence measurement window 530, the sample moves to the end point C of the fluorescence measurement window 530. The speed of the lateral flow is calculated by detecting lateral flow end time of the sample at the end point C of the fluorescence measurement window 530.

FIG. 10 is an example in the case where the two light sources 220 and 225 and the two light detectors 230 and 235 are provided in an embodiment of the present disclosure. However, a similar operation is performed only when one light source or one light detector is present. In this case, a movement distance of the optical module 200 may be further increased.

Hereinafter, a displacement of the optical module 200 by the driving module 300 is described.

As described above, the driving carrier 330 is fixed to the module lower part 214 of the optical module 200. The driving carrier 330 and the driving shaft 320 are connected. When the driving shaft 320 is rotated by a rotatory power of the motor 310, the driving carrier 330 is displaced in the front and back direction. The optical module 200 coupled to the driving carrier 330 may be displaced in the front and back direction.

Due to such a configuration, a displacement distance between the carrier 330 and the optical module 200 can be controlled by the number of rotations of the motor 310, so that accurate control over a displacement distance of the optical module 200 can be performed. Furthermore, as described above, motive power can be effectively delivered because the carrier 330 can be strongly attached to the driving shaft 320, and accurate control over the displacement distance can be more effectively achieved. Moreover, the detachment, out-of phase, etc. of the optical module 200 can be prevented because the guide shafts 352 and 354 are further provided to stably guide a displacement of the optical module 200.

The immunodiagnostic fluorescence reader1 according to the present application comprises an optimal construction for an implementation of the aforementioned method, and may be implemented as hardware, firmware, or software for the implementation of the method or a combination of them. If the method is implemented as software, a storage medium comprises a given medium for storage or delivery in a form readable by a device, such as a computer. For example, the computer-readable medium comprises a read only memory (ROM); a random access memory (RAM); a magnetic disk storage medium; an optical storage medium; a flash memory device, other electrical, optical, or acoustic signal delivery media, etc.

Furthermore, although the preferred embodiments have been illustrated and described above, this specification is not limited to the aforementioned specific embodiments, and a person having ordinary skill in the art to which this specification pertains may modify the present disclosure in various ways without departing from the scope of the appended claims.

**[Description of reference Numerals]**

| | | | |
|---|---|---|---|
| 1: | fluorescence reader for immunodiagnosis | | |
| 100: | base frame | 110: | entrance part on side at front |
| 120: | inner space | 130: | cartridge fixing part |
| 131: | fixing spring | 132: | lifting-up protrusion |
| 134: | side fitting frame | 140: | guide shaft mounting groove |
| 145: | guide shaft fitting groove | 150: | upper frame |
| 152: | guide shaft fixing protrusion | 155: | display part |
| 200: | optical module | 212: | module upper plate |
| 214: | module lower plate | 216: | first guide protrusion |
| 217: | second guide protrusion | 219: | cylinder barrel |
| 220: | light source | 222: | light source board for fluorescence |
| 223: | light source board for barcode | 225: | barcode light source |
| 230: | fluorescence detector | 235: | ambient light detector |
| 241: | lens for light source | 242: | first mirror |
| 244: | second mirror | 246: | downward lens |
| 254: | detection lens | 256: | pin hole |
| 260: | interrupt protrusion part | 300: | driving module |
| 310: | motor | 320: | driving shaft |
| 330: | driving carrier | 352: | first guide shaft |
| 354: | second guide shaft | 400: | reference position detection module |
| 410: | module main board | 420: | interrupt sensor part |
| 425: | inserted sensor | 430: | cartridge sensing switch |
| 500: | diagnostic cartridge | 510: | barcode |
| 520: | sample inlet | 530: | fluorescence measurement window |
| 540: | first end | 550: | second end |
| 560: | bottom groove | | |

## Claims

1. An integrated immunodiagnostic fluorescence reader (1) comprising:
a base frame (100) with an open top surface, comprising:
an entrance part (110) at a front side thereof into which a diagnostic cartridge (500) having a barcode (510) differently assigned to each diagnosis marker and a fluorescence measurement window (530) is insertable, wherein the barcode (510) is located on the same side as the fluorescence measurement window (530);
an insertion space (120) opened to an outside through the entrance part (110), wherein the cartridge is insertable through the entrance part (110);
a space for the movement of an optical module (200), being located on an upper part of the insertion space (120) in which the optical module (200) is configured to move;
a space for driving module mounting, being located on one side of the space for the movement of the optical module (200);
a space for reference position detection module mounting; and
a cartridge fixing part (130) configured for fixing the diagnostic cartridge (500) at a position by applying pressure to at least part of the diagnostic cartridge (500) when the diagnostic cartridge (500) is inserted into the insertion space (120);
the optical module (200) located in the space for the movement of the optical module (200) of the base frame (100);
a driving module (300) located in the space for driving module mounting of the base frame (100) and connected to the optical module (200) to move the optical module (200) , wherein the driving module (300) includes two guide shafts (352, 354), each fixed to the base frame (100) in parallel to a lengthwise direction of the diagnostic cartridge (500) located in the insertion space (120), wherein one end of each of the two guide shafts (352, 354) is fit and fixed in fitting grooves (145) of the base frame (100) and the other end of each of the two guide shafts (352, 354) is mounted in mounting grooves (140) of the base frame (100);
wherein the optical module (200) is configured to move along the two guide shafts (352, 354) and to radiate light to the fluorescence measurement window (530) and the barcode (510) of the diagnostic cartridge (500) while moving in parallel to the lengthwise direction of the diagnostic cartridge (500) along the two guide shafts (352, 354) and to detect fluorescence light and reflected light reflected from the barcode (510);
a detection module (400) for a reference position located in the space for reference position detection module mounting of the base frame (100) and configured to detect a reference position of the optical module (200) and to detect whether the cartridge (500) is inserted; and
an upper frame (150) configured to cover and fix the opened top surface of the base frame (100) comprising a guide shaft fixing protrusion (152) fixing one end of each of the guide shafts (352, 354) together with a guide shaft mounting groove (140) of the base frame (100) and a fastening part fastened to the base frame (100) in order to maintain the fixing by the guide shaft fixing protrusion (152).

2. The integrated immunodiagnostic fluorescence reader (1) of claim 1, wherein the optical module (200) comprises:
a module casing comprising a module lower part (214) and a module upper plate (212) covering and fixing an opened top surface of the module lower part (214) to form an box shaped internal space;
a light source (220, 225) disposed within the module casing;
a light guide part disposed within the module casing and configured to guide a light from the light source (225) to the barcode (510) of the diagnostic cartridge (500) and to guide a reflected light reflected from the barcode (510) to a light detector (235);
a fluorescence guide part disposed within the module casing comprising a dichroic mirror for guiding a light from the light source (220) to the fluorescence measurement window (530) of the diagnostic cartridge (500) and for guiding a fluorescence emitted from the fluorescence measurement window (530) to a light detector (230); and
a light detector (230, 235) configured to detect the reflected light and the fluorescence;
wherein the module lower part (214) on one side thereof has a cylinder barrel (219) into which one of the two guide shafts (352, 354) is put in to guide the optical module (200) in a lengthwise direction, and on the other side thereof has guide protrusions (216) and (217) to guide the optical module (200) along the other of the two guide shafts (352, 354), and on the bottom side thereof has an interrupt protrusion part (260) being inserted into the detection module (400) for a reference position to determine the reference position.

3. The integrated immunodiagnostic fluorescence reader (1) of claim 2, wherein the fluorescence guide part comprises:
a lens for light source (241) for concentrating irradiation light emitted from the light source (220) in a horizontal lateral direction;
a dichroic first mirror (242) for changing a path of the irradiation light concentrated by the lens for light source (241) so that the irradiation light travels in a horizontal and front direction, and for transmitting straight the fluorescence emitted from the fluorescence measurement window (530) of the diagnostic cartridge (500);
a second mirror (244) for changing a path of the light whose path has been changed by the dichroic first mirror (242) in a vertical and downward direction to send the light to the fluorescence measurement window (530) of the diagnostic cartridge (500), and for sending the fluorescence to the first mirror (242), generated from the fluorescence measurement window (530) of the diagnostic cartridge (500);
a downward lens (246) for concentrating the light whose path has been changed by the second mirror (244), irradiating the concentrated light to the fluorescence measurement window (530) of the diagnostic cartridge (500), concentrating the fluorescence emitted from the fluorescence measurement window (530), and sending the concentrated fluorescence to the second mirror (244);
a detection lens (254) for concentrating the fluorescence whose path has been changed by the second mirror (244) and transmitted straight through the first mirror (242), and concentrated by the downward lens (246); and
a pin hole (256) for filtering, transmitting and guiding the fluorescence concentrated by the detection lens (254).

4. The integrated immunodiagnostic fluorescence reader (1) of claim 3,
wherein the light source (220) is one,
wherein the light guide part shares a path of light incident on the barcode (510) with the path of the fluorescence guide part, and further comprises a mirror for adjusting the path of light incident on the barcode (510) or a path of light reflected from the barcode (510) so that the reflected light reflected from the barcode (510) is perpendicularly incident on the light detector (235).

5. The immunodiagnostic fluorescence reader (1) of claim 4,
wherein the light detector (230, 235) comprises a light detector (235) and a fluorescence detector (230),
wherein the light detector (235) is disposed on the path of the light emitted from the barcode (510) so that the reflected light reflected from the barcode (510) is perpendicularly incident on the light detector (235).

6. The integrated immunodiagnostic fluorescence reader (1) of claim 3,
wherein the light sources (220, 225) are two,
wherein the path of the light incident on the barcode (510) is different in the light guide part and in the fluorescence guide part,
wherein each light emitted by the two light sources (220, 225) is guided to the detectors (230, 235) along the light guide part and along the fluorescence guide part, respectively,
wherein a light source of the light incident on the light guide part is disposed adjacent to the light detector so that the reflected light reflected from the barcode (510) is perpendicularly incident on the light detector.

7. The immunodiagnostic fluorescence reader (1) of claim 6,
wherein the light detector comprises the light detector (235) and the fluorescence detector (230),
wherein the light source of the light incident on the light guide part and the light detector are adjacently disposed so that the reflected light reflected from the barcode (510) is perpendicularly incident on the light detector.

8. The integrated immunodiagnostic fluorescence reader (1) of claim 1, wherein the driving module (300) comprises:
a motor (310) configured to provide a rotatory power;
a driving shaft (320) connected to the motor (310) in a way to rotate and disposed on at least one lateral side of the optical module (200), and extending in a front and back direction; and
a driving carrier (330) disposed between the optical module (200) and the driving shaft (320) and connected to the optical module (200) and the driving shaft (320),
wherein when the motor (310) rotates and thus the driving shaft (320) is rotated, the driving carrier (330) is displaced in the front and back direction, the two guide shafts (352, 354) are configured to guide the optical module (200) by a cylinder barrel (219) and guide protrusions (216, 217), and the optical module (200) connected to the driving carrier (330) is guided by the two guide shafts (352, 354) and displaced in the front and back direction.

9. The integrated immunodiagnostic fluorescence reader (1) of claim 1, wherein the cartridge fixing part (130) of the base frame (100) comprises:
a fixing spring (131) configured to lift up and fix a bottom of the diagnostic cartridge (500);
a lifting-up protrusion (132) configured to be matched with and fix a portion of the bottom groove (560) of the diagnostic cartridge (500); and
a side fitting frame (134) configured to fix the diagnostic cartridge (500) by inserting a side of the diagnostic cartridge (500) therein.

10. The integrated immunodiagnostic fluorescence reader (1) of claim 2, wherein the reference position detection module (400) comprises:
a module main board (410) fixed in a direction opposite to the entrance part (110) of the base frame (100); and
an interrupt sensor part (420) disposed in a direction opposite to the entrance part (110) on the module main board (410), wherein the interrupt protrusion part (260) is inserted into the interrupt sensor part (420),
wherein a position of the optical module (200) is displaced between the reference position where the interrupt protrusion part (260) is inserted into the interrupt sensor part (420) and a position of the entrance part (110).

11. The integrated immunodiagnostic fluorescence reader (1) of claim 10, wherein the reference position detection module (400) further comprises a cartridge sensing switch (430) disposed under the module main board (410),
wherein the diagnostic cartridge (500) is configured to press the cartridge sensing switch (430) when the diagnostic cartridge (500) is inserted into the base frame (100) through the entrance part (110) and an end of the diagnostic cartridge (500) reaches a specific position, and
wherein the cartridge sensing switch (430) generates an insertion signal for the diagnostic cartridge (500) when the diagnostic cartridge (500) presses the cartridge sensing switch (430).

12. The integrated immunodiagnostic fluorescence reader (1) of claim 1, wherein the diagnostic cartridge (500) comprises the fluorescence measurement window (530) extended in a lateral flow direction of a sample and having the lateral flow of the sample exposed upward, and a barcode (510) for identifying a type of sample,
wherein the optical module (200) is configured to:
identify the type of sample by reading the barcode (510) by moving to the barcode area,
move to an end point (C) of the fluorescence measurement window (530) after detecting lateral flow start time of the sample at a start point (B) of the fluorescence measurement window (530), and
calculate a flow rate of the lateral flow by detecting lateral flow end time of the sample at the end point (C) of the fluorescence measurement window (530).

13. The immunodiagnostic fluorescence reader (1) of claim 6 or 7, wherein the diagnostic cartridge (500) comprises the fluorescence measurement window (530) extended in a lateral flow direction of a sample and having the lateral flow of the sample exposed upward, and a barcode (510),
wherein the optical module (200) is configured to:
identify the type of sample by reading the barcode (510) by moving for the barcode light source (225) to irradiate a light to the barcode (510);
move to an end point (C) of the fluorescence measurement window (530) after detecting lateral flow start time of the sample at a start point (B) of the fluorescence measurement window (530), and
calculate a flow rate of the lateral flow by detecting lateral flow end time of the sample at the end point (C) of the fluorescence measurement window (530).

## Patentansprüche

1. Integriertes immundiagnostisches Fluoreszenzlesegerät (1), das umfasst:
einen Basisrahmen (100) mit einer offenen oberen Fläche, der umfasst:
einen Eingangsabschnitt (110) an seiner Vorderseite, in den eine Diagnosekassette (500) mit einem Barcode (510), der jeder Diagnosemarkierung unterschiedlich zugewiesen wird, und einem Fluoreszenzmessfenster (530) eingeführt werden kann, wobei sich der Barcode (510) auf derselben Seite wie das Fluoreszenzmessfenster (530) befindet;
einen Einführraum (120), der durch den Eingangsabschnitt (110) zu einer Außenseite geöffnet ist, wobei die Kassette durch den Eingangsabschnitt (110) einführbar ist;
einen Raum für die Bewegung eines optischen Moduls (200), der sich auf einem oberen Teil des Einführraums (120) befindet, in dem das optische Modul (200) konfiguriert ist, sich zu bewegen;
einen Raum für die Anbringung eines Antriebsmoduls, der sich auf einer Seite des Raums für die Bewegung des optischen Moduls (200) befindet;
einen Raum für die Anbringung eines Referenzpositionsdetektionsmoduls; und
einen Kassettenfixierungsabschnitt (130), der konfiguriert ist, die Diagnosekassette (500) durch Ausüben eines Drucks auf mindestens einen Teil der Diagnosekassette (500) an einer Position zu fixieren, wenn die Diagnosekassette (500) in den Einführraum (120) eingeführt wird;
das optische Modul (200), das sich in dem Raum für die Bewegung des optischen Moduls (200) des Basisrahmens (100) befindet;
ein Antriebsmodul (300), das sich in dem Raum für eine Anbringung eines Antriebsmoduls des Basisrahmens (100) befindet und mit dem optischen Modul (200) verbunden ist, um das optische Modul (200) zu bewegen, wobei das Antriebsmodul (300) zwei Führungswellen (352, 354) enthält, die jeweils an dem Basisrahmen (100) parallel zu einer Richtung der Länge der Diagnosekassette (500), die sich in dem Einführraum (120) befindet, fixiert ist, wobei ein Ende jeder der beiden Führungswellen (352, 354) eingepasst ist und in Passnuten (145) des Basisrahmens (100) angebracht ist und das andere Ende jeder der beiden Führungswellen (352, 354) in Anbringungsnuten (140) des Basisrahmens (100) angebracht ist;
wobei das optische Modul (200) konfiguriert ist, sich entlang der beiden Führungswellen (352, 354) zu bewegen und Licht auf das Fluoreszenzmessfenster (530) und den Barcode (510) zu strahlen, während es sich parallel zu der Richtung der Länge der Diagnosekassette (500) entlang der beiden Führungswellen (352, 354) bewegt, und Fluoreszenzlicht und reflektiertes Licht, das von dem Barcode (510) reflektiert wird, zu detektieren;
ein Detektionsmodul (400) für eine Referenzposition, das sich in dem Raum für die Anbringung eines Referenzpositionsdetektionsmoduls des Basisrahmens (100) befindet und konfiguriert ist, eine Referenzposition des optischen Moduls (200) zu detektieren und zu detektieren, ob die Kassette (500) eingeführt wurde; und
einen oberen Rahmen (150), der konfiguriert ist, die geöffnete obere Fläche des Basisrahmens (100) zu bedecken und zu fixieren, der einen Führungswellenfixierungsvorsprung (152) umfasst, der ein Ende jeder der Führungswellen (352, 354) zusammen mit einer Führungswellenanbringungsnut (140) des Basisrahmens (100) und einen Anbringungsteil, der an dem Basisrahmen (100) befestigt ist, fixiert, um die Fixierung durch den Führungswellenfixierungsvorsprung (152) beizubehalten.

2. Integriertes immundiagnostisches Fluoreszenzlesegerät (1) nach Anspruch 1, wobei das optische Modul (200) umfasst:
ein Modulgehäuse, das einen unteren Modulteil (214) und eine obere Modulplatte (212), die eine geöffnete obere Fläche des unteren Modulteils (214) bedeckt und fixiert, um einen kastenförmigen inneren Raum zu bilden, umfasst;
eine Lichtquelle (220, 225), die innerhalb des Modulgehäuses angeordnet ist;
einen Lichtführungsabschnitt, der innerhalb des Modulgehäuses angeordnet ist und konfiguriert ist, ein Licht von der Lichtquelle (225) zu dem Barcode (510) der Diagnosekassette (500) zu lenken und ein reflektiertes Licht, das von dem Barcode (510) reflektiert wird, zu einem Lichtdetektor (235) zu lenken;
einen Fluoreszenzführungsabschnitt, der innerhalb des Modulgehäuses angeordnet ist, und einen dichroitischen Spiegel zum Lenken eines Lichts von der Lichtquelle (220) zu dem Fluoreszenzmessfenster (530) der Diagnosekassette (500) und zum Lenken einer Fluoreszenz, die von dem Fluoreszenzmessfenster (530) ausgesendet wird, zu einem Lichtdetektor (230) umfasst; und
einen Lichtdetektor (230, 235), der konfiguriert ist, das reflektierte Licht und die Fluoreszenz zu detektieren;
wobei der untere Modulteil (214) auf einer Seite von ihm ein Zylinderlager (219) besitzt, in das eine der beiden Führungswellen (352, 354) eingeführt wird, um das optische Modul (200) in eine Richtung der Länge zu lenken, und auf der anderen Seite von ihm Führungsvorsprünge (216) und (217) besitzt, um das optische Modul (200) entlang der anderen der beiden Führungswellen (352, 354) zu lenken, und auf der unteren Seite von ihm einen Unterbrechungsvorsprungsabschnitt (260) besitzt, der in das Referenzpositionsdetektionsmodul (400) eingeführt wird, um die Referenzposition zu bestimmen.

3. Integriertes immundiagnostisches Fluoreszenzlesegerät (1) nach Anspruch 2, wobei der Fluoreszenzführungsabschnitt umfasst:
eine Linse für eine Lichtquelle (241) zum Bündeln von Strahlungslicht, das von der Lichtquelle (220) ausgestrahlt wird, in einer horizontalen seitlichen Richtung;
einen dichroitischen ersten Spiegel (242), um einen Weg des Strahlungslichts, das durch die Linse für eine Lichtquelle (241) gebündelt wird, so zu ändern, dass das Strahlungslicht in einer horizontalen Vorwärtsrichtung läuft, und um die Fluoreszenz, die von dem Fluoreszenzmessfenster (530) der Diagnosekassette (500) ausgesendet wird, geradeaus zu senden;
einen zweiten Spiegel (244), um einen Weg des Lichts, dessen Weg durch den dichroitischen ersten Spiegel (242) geändert wurde, in einer vertikalen Abwärtsrichtung zu ändern, um das Licht zu dem Fluoreszenzmessfenster (530) der Diagnosekassette (500) zu senden und um die Fluoreszenz, die von dem Fluoreszenzmessfenster (530) der Diagnosekassette (500) erzeugt wird, zu dem ersten Spiegel (242) zu senden;
eine Abwärtslinse (246) zum Bündeln des Lichts, dessen Weg durch den zweiten Spiegel (244) geändert wurde, Strahlen des gebündelten Lichts auf das Fluoreszenzmessfenster (530) der Diagnosekassette (500), Bündeln der von dem Fluoreszenzmessfenster (530) ausgeendeten Fluoreszenz und Senden der gebündelten Fluoreszenz zu dem zweiten Spiegel (244);
eine Detektionslinse (254) zum Bündeln der Fluoreszenz, dessen Weg durch den zweiten Spiegel (244) geändert und geradeaus durch den ersten Spiegel (242) gesendet und durch die Abwärtslinse (246) gebündelt wurde; und
ein Stiftloch (256) zum Filtern, Senden und Lenken der durch die Detektionslinse (254) gebündelten Fluoreszenz.

4. Integriertes immundiagnostisches Fluoreszenzlesegerät (1) nach Anspruch 3,
wobei die Lichtquelle (220) eine ist,
wobei der Lichtführungsabschnitt einen Weg des Lichts, das auf den Barcode (510) einfällt, mit dem Weg des Fluoreszenzführungsabschnitts (510) gemeinsam nutzt und ferner einen Spiegel umfasst, um den Weg des Lichts, das auf den Barcode (510) einfällt, oder einen Weg des von dem Barcode (510) reflektierten Lichts anzupassen, so dass das reflektierte Licht, das von dem Barcode (510) reflektiert wird, senkrecht auf den Lichtdetektor (235) einfällt.

5. Immundiagnostisches Fluoreszenzlesegerät (1) nach Anspruch 4,
wobei der Lichtdetektor (230, 235) einen Lichtdetektor (235) und einen Fluoreszenzdetektor (230) umfasst,
wobei der Lichtdetektor (235) auf dem Weg des von dem Barcode (510) ausgesendeten Lichts angeordnet ist, so dass das reflektierte Licht, das von dem Barcode (510) reflektiert wird, senkrecht auf den Lichtdetektor (235) einfällt.

6. Integriertes immundiagnostisches Fluoreszenzlesegerät (1) nach Anspruch 3,
wobei zwei der Lichtquellen (220, 225) vorhanden sind,
wobei der Weg des Lichts, das auf den Barcode (510) einfällt, in dem Lichtführungsabschnitt und in dem Fluoreszenzführungsabschnitt verschieden ist,
wobei jedes durch die beiden Lichtquellen (220, 225) ausgesendete Licht jeweils entlang des Lichtführungsabschnitts und entlang des Fluoreszenzführungsabschnitts gelenkt wird,
wobei eine Lichtquelle des auf den Lichtführungsabschnitt einfallenden Lichts benachbart zu dem Lichtdetektor angeordnet ist, so dass das reflektierte Licht, das von dem Barcode (510) reflektiert wird, senkrecht auf den Lichtdetektor einfällt.

7. Immundiagnostisches Fluoreszenzlesegerät (1) nach Anspruch 6,
wobei der Lichtdetektor den Lichtdetektor (235) und den Fluoreszenzdetektor (230) umfasst,
wobei die Lichtquelle des auf den Lichtführungsabschnitt einfallenden Lichts und der Lichtdetektor benachbart angeordnet sind, so dass das reflektierte Licht, das von dem Barcode (510) reflektiert wird, senkrecht auf den Lichtdetektor einfällt.

8. Integriertes immundiagnostisches Lesegerät (1) nach Anspruch 1, wobei das Antriebsmodul (300) umfasst:
einen Motor (310), der konfiguriert ist, Drehleistung zu liefern;
eine Antriebswelle (320), die mit dem Motor (310) in einer Weise verbunden ist, dass sie sich dreht, und auf mindestens einer seitlichen Seite des optischen Moduls (200) angeordnet ist und sich in einer Vorwärts/Rückwärts-Richtung erstreckt; und
einen Antriebsträger (330), der zwischen dem optischen Modul (200) und der Antriebswelle (320) angeordnet ist und mit dem optischen Modul (200) und der Antriebswelle (320) verbunden ist,
wobei dann, wenn sich der Motor (310) dreht und dadurch die Antriebswelle gedreht wird, der Antriebsträger (330) in der Vorwärts/Rückwärts-Richtung verschoben wird, wobei die zwei Führungswellen (352, 354) konfiguriert sind, das optische Modul (200) durch ein Zylinderlager (219) und Führungsvorsprünge (216, 217) zu lenken und das optische Modul (200), das mit dem Antriebsträger (330) verbunden ist, durch die beiden Führungswellen (352, 354) gelenkt wird und in der Vorwärts/Rückwärts-Richtung verschoben wird.

9. Integriertes immundiagnostisches Fluoreszenzlesegerät (1) nach Anspruch 1, wobei der Kassettenfixierungsabschnitt (130) des Basisrahmens (100) umfasst:
eine Fixierungsfeder (131), die konfiguriert ist, einen Boden der Diagnosekassette (500) anzuheben und zu fixieren;
einen Anhebungsvorsprung (132), der konfiguriert ist, mit einem Teil der unteren Nut (560) der Diagnosekassette (500) zusammenzupassen und diese anzubringen; und
einen Seiteneinpassungsrahmen (134), der konfiguriert ist, die Diagnosekassette (500) durch Einführen einer Seite der Diagnosekassette (500) in sie anzubringen.

10. Integriertes immundiagnostisches Fluoreszenzlesegerät (1) nach Anspruch 2, wobei das Referenzpositionsdetektionsmodul (400) umfasst:
eine Modulhauptkarte (410), die in einer Richtung entgegengesetzt zu dem Eingangsabschnitt (110) des Basisrahmens (100) fixiert ist; und
einen Unterbrechungssensorabschnitt (420), der in einer Richtung entgegengesetzt zu dem Eingangsabschnitt (110) auf der Modulhauptkarte (410) angeordnet ist, wobei der Unterbrechungsvorsprungsabschnitt (260) in den Unterbrechungssensorabschnitt (420) eingeführt ist,
wobei eine Position des optischen Moduls (200) zwischen der Referenzposition, wo der Unterbrechungsvorsprungsabschnitt (260) in den Unterbrechungssensorabschnitt (420) eingeführt ist, und einer Position des Eingangsabschnitts (110) verschoben wird.

11. Integriertes immundiagnostisches Fluoreszenzlesegerät (1) nach Anspruch 10, wobei das Referenzpositionsdetektionsmodul (400) ferner einen Kassettenerfassungsschalter (430) umfasst, der unter der Modulhauptkarte (410) angeordnet ist,
wobei die Diagnosekassette (500) konfiguriert ist, den Kassettenerfassungsschalter (430) zu drücken, wenn die Diagnosekassette (500) in den Basisrahmen (100) durch den Eingangsabschnitt (110) eingeführt wird und ein Ende der Diagnosekassette (500) eine spezifische Position erreicht, und
wobei der Kassettenerfassungsschalter (430) ein Einführungssignal für die Diagnosekassette (500) erzeugt, wenn die Diagnosekassette (500) den Kassettenerfassungsschalter (430) drückt.

12. Integriertes immundiagnostisches Fluoreszenzlesegerät (1) nach Anspruch 1, wobei die Diagnosekassette (500) das Fluoreszenzmessfenster (530), das sich in einer seitlichen Flussrichtung einer Probe erstreckt, wobei der seitliche Fluss der Probe nach oben freiliegt, und einen Barcode (510) zum Identifizieren eines Probentyps umfasst,
wobei das optische Modul (200) konfiguriert ist zum:
Identifizieren des Probentyps durch Lesen des Barcodes (510) durch Bewegen des Barcodebereichs,
Bewegen zu einem Endpunkt (C) des Fluoreszenzmessfensters (530) nach dem Detektieren einer Startzeit des seitlichen Flusses der Probe an einem Startpunkt (B) des Fluoreszenzmessfensters (530) und
Berechnen einer Flussrate des seitlichen Flusses durch Detektieren einer Endzeit des seitlichen Flusses der Probe an dem Endpunkt (C) des Fluoreszenzmessfensters (530).

13. Immundiagnostisches Fluoreszenzlesegerät (1) nach Anspruch 6 oder 7, wobei die Diagnosekassette (500) das Fluoreszenzmessfenster (530), dass sich in einer Richtung eines seitlichen Flusses einer Probe erstreckt, wobei der seitliche Fluss der Probe nach oben freiliegt, und einen Barcode (510) umfasst,
wobei das optische Modul (200) konfiguriert ist zum:
Identifizieren des Probentyps durch Lesen des Barcodes (510) durch Bewegen, damit die Barcodelichtquelle (225) Licht auf den Barcode (510) strahlt;
Bewegen zu einem Endpunkt (C) des Fluoreszenzmessfensters (530) nach dem Detektieren einer Startzeit eines seitlichen Flusses der Probe an einem Startpunkt (B) des Fluoreszenzmessfensters (530) und
Berechnen einer Flussrate des seitlichen Flusses durch Detektieren einer Endzeit des seitlichen Flusses der Probe an einem Endpunkt (C) des Fluoreszenzmessfensters (530).

## Revendications

1. Lecteur de fluorescence d'immunodiagnostic intégré (1) comportant :
un châssis de base (100) avec une surface supérieure ouverte, comportant :
une partie d'entrée (110) sur un côté avant de celui-ci, dans laquelle une cartouche de diagnostic (500) ayant un code à barres (510) affecté différemment à chaque marqueur de diagnostic et une fenêtre de mesure de fluorescence (530) peut être insérée, dans lequel le code à barres (510) est situé du même côté que la fenêtre de mesure de fluorescence (530) ;
un espace d'insertion (120) ouvert vers un extérieur à travers la partie d'entrée (110), dans lequel la cartouche peut être insérée à travers la partie d'entrée (110) ;
un espace pour le mouvement d'un module optique (200), étant situé sur une partie supérieure de l'espace d'insertion (120) dans lequel le module optique (200) est configuré pour se déplacer ;
un espace de montage de module d'entraînement, étant situé sur un côté de l'espace pour le mouvement du module optique (200) ;
un espace de montage de module de détection de position de référence ; et
une partie de fixation de cartouche (130) configurée pour fixer la cartouche de diagnostic (500) à une position en appliquant une pression sur au moins une partie de la cartouche de diagnostic (500) lorsque la cartouche de diagnostic (500) est insérée dans l'espace d'insertion (120) ;
le module optique (200) situé dans l'espace pour le mouvement du module optique (200) du châssis de base (100) ;
un module d'entraînement (300) situé dans l'espace de montage de module d'entraînement du châssis de base (100) et relié au module optique (200) pour déplacer le module optique (200), dans lequel le module d'entraînement (300) inclut deux tiges de guidage (352, 354), chacune fixée au châssis de base (100) parallèlement à une direction longitudinale de la cartouche de diagnostic (500) située dans l'espace d'insertion (120), dans lequel une extrémité de chacune des deux tiges de guidage (352, 354) est ajustée et fixée dans des rainures d'ajustement (145) du châssis de base (100) et l'autre extrémité de chacune des deux tiges de guidage (352, 354) est montée dans des rainures de montage (140) du châssis de base (100) ;
dans lequel le module optique (200) est configuré pour se déplacer le long des deux tiges de guidage (352, 354) et pour rayonner de la lumière vers la fenêtre de mesure de fluorescence (530) et le code à barres (510) de la cartouche de diagnostic (500) tout en se déplaçant parallèlement à la direction longitudinale de la cartouche de diagnostic (500) le long des deux tiges de guidage (352, 354) et pour détecter de la lumière de fluorescence et de la lumière réfléchie réfléchie par le code à barres (510) ;
un module de détection (400) pour une position de référence située dans l'espace de montage de module de détection de position de référence du châssis de base (100) et configuré pour détecter une position de référence du module optique (200) et pour détecter si la cartouche (500) est insérée ; et
un châssis supérieur (150) configuré pour recouvrir et fixer la surface supérieure ouverte du châssis de base (100) comportant une saillie de fixation de tige de guidage (152) fixant une extrémité de chacune des tiges de guidage (352, 354) en association avec une rainure de montage de tige de guidage (140) du châssis de base (100) et une partie de fixation fixée au châssis de base (100) afin de maintenir la fixation par la saillie de fixation de tige de guidage (152).

2. Lecteur de fluorescence d'immunodiagnostic intégré (1) selon la revendication 1, dans lequel le module optique (200) comporte :
un boîtier de module comportant une partie inférieure de module (214) et une plaque supérieure de module (212) recouvrant et fixant une surface supérieure ouverte de la partie inférieure de module (214) pour former un espace interne en forme de boîte ;
une source de lumière (220, 225) disposée à l'intérieur du boîtier de module ;
une partie de conduction de lumière disposée à l'intérieur du boîtier de module et configurée pour conduire une lumière provenant de la source de lumière (225) jusqu'au code à barres (510) de la cartouche de diagnostic (500) et pour conduire une lumière réfléchie réfléchie par le code à barres (510) jusqu'à un détecteur de lumière (235) ;
une partie de conduction de fluorescence disposée à l'intérieur du boîtier de module comportant un miroir dichroïque pour conduire une lumière de la source de lumière (220) jusqu'à la fenêtre de mesure de fluorescence (530) de la cartouche de diagnostic (500) et pour conduire une fluorescence émise à partir de la fenêtre de mesure de fluorescence (530) jusqu'à un détecteur de lumière (230) ; et
un détecteur de lumière (230, 235) configuré pour détecter la lumière réfléchie et la fluorescence ;
dans lequel la partie inférieure de module (214) a, sur un côté de celle-ci, un barillet cylindrique (219) dans lequel l'une des deux tiges de guidage (352, 354) est insérée pour guider le module optique (200) dans une direction longitudinale, et a, de l'autre côté de celle-ci, des saillies de guidage (216) et (217) pour guider le module optique (200) le long de l'autre des deux tiges de guidage (352, 354), et a, sur le côté inférieur de celle-ci, une partie de saillie d'interruption (260) étant insérée dans le module de détection (400) pour une position de référence afin de déterminer la position de référence.

3. Lecteur de fluorescence d'immunodiagnostic intégré (1) selon la revendication 2, dans lequel la partie de conduction de fluorescence comporte :
une lentille pour source de lumière (241) pour concentrer de la lumière d'irradiation émise à partir de la source de lumière (220) dans une direction latérale horizontale ;
un premier miroir dichroïque (242) pour changer un trajet de la lumière d'irradiation concentrée par la lentille pour source de lumière (241) de sorte que la lumière d'irradiation se propage dans une direction horizontale et vers l'avant, et à transmettre directement la fluorescence émise à partir de la fenêtre de mesure de fluorescence (530) de la cartouche de diagnostic (500) ;
un second miroir (244) pour changer un trajet de la lumière dont le trajet a été changé par le premier miroir dichroïque (242) dans une direction verticale et vers le bas pour envoyer la lumière vers la fenêtre de mesure de fluorescence (530) de la cartouche de diagnostic (500), et pour envoyer la fluorescence vers le premier miroir (242), générée à partir de la fenêtre de mesure de fluorescence (530) de la cartouche de diagnostic (500) ;
une lentille dirigée vers le bas (246) pour concentrer la lumière dont le trajet a été changé par le second miroir (244), rayonner la lumière concentrée vers la fenêtre de mesure de fluorescence (530) de la cartouche de diagnostic (500), concentrer la fluorescence émise à partir de la fenêtre de mesure de fluorescence (530) et envoyer la fluorescence concentrée vers le second miroir (244) ;
une lentille de détection (254) pour concentrer la fluorescence dont le trajet a été changé par le second miroir (244) et transmise directement à travers le premier miroir (242), et concentrée par la lentille dirigée vers le bas (246) ; et
un trou d'épingle (256) pour filtrer, transmettre et guider la fluorescence concentrée par la lentille de détection (254).

4. Lecteur de fluorescence d'immunodiagnostic intégré (1) selon la revendication 3,
dans lequel la source de lumière (220) est au nombre de un,
dans lequel la partie de conduction de lumière partage un trajet de lumière incidente sur le code à barres (510) avec le trajet de la partie de conduction de fluorescence, et comporte en outre un miroir pour régler le trajet de la lumière incidente sur le code à barres (510) ou un trajet de la lumière réfléchie par le code à barres (510) de sorte que la lumière réfléchie réfléchie à partir du code à barres (510) est perpendiculairement incidente sur le détecteur de lumière (235).

5. Lecteur de fluorescence d'immunodiagnostic (1) selon la revendication 4,
dans lequel le détecteur de lumière (230, 235) comporte un détecteur de lumière (235) et un détecteur de fluorescence (230),
dans lequel le détecteur de lumière (235) est disposé sur le trajet de la lumière émise à partir du code à barres (510) de sorte que la lumière réfléchie réfléchie par le code à barres (510) est perpendiculairement incidente sur le détecteur de lumière (235).

6. Lecteur de fluorescence d'immunodiagnostic intégré (1) selon la revendication 3,
dans lequel les sources de lumière (220, 225) sont au nombre de deux,
dans lequel le trajet de la lumière incidente sur le code à barres (510) est différent dans la partie de conduction de lumière et dans la partie de conduction de fluorescence,
dans lequel chaque lumière émise par les deux sources de lumière (220, 225) est conduite jusqu'aux détecteurs (230, 235) le long de la partie de conduction de lumière et le long de la partie de conduction de fluorescence, respectivement,
dans lequel une source de lumière de la lumière incidente sur la partie de conduction de lumière est disposée au voisinage du détecteur de lumière de sorte que la lumière réfléchie réfléchie par le code à barres (510) est incidente perpendiculairement sur le détecteur de lumière.

7. Lecteur de fluorescence d'immunodiagnostic (1) selon la revendication 6,
dans lequel le détecteur de lumière comporte le détecteur de lumière (235) et le détecteur de fluorescence (230),
dans lequel la source de lumière de la lumière incidente sur la partie de conduction de lumière et le détecteur de lumière sont disposés de manière adjacente de sorte que la lumière réfléchie réfléchie par le code à barres (510) est perpendiculairement incidente sur le détecteur de lumière.

8. Lecteur de fluorescence d'immunodiagnostic intégré (1) selon la revendication 1, dans lequel le module d'entraînement (300) comporte :
un moteur (310) configuré pour fournir une puissance de rotation ;
un arbre d'entraînement (320) relié au moteur (310) de manière à tourner et disposé sur au moins un côté latéral du module optique (200), et s'étendant dans une direction avant et arrière ; et
un support d'entraînement (330) disposé entre le module optique (200) et l'arbre d'entraînement (320) et relié au module optique (200) et à l'arbre d'entraînement (320),
dans lequel lorsque le moteur (310) tourne et entraîne ainsi l'arbre d'entraînement (320) en rotation, le support d'entraînement (330) est déplacé dans la direction avant et arrière, les deux tiges de guidage (352, 354) sont configurées pour guider le module optique (200) par un fût cylindrique (219) et des saillies de guidage (216, 217), et le module optique (200) relié au support d'entraînement (330) est guidé par les deux tiges de guidage (352, 354) et déplacé dans la direction avant et arrière.

9. Lecteur de fluorescence d'immunodiagnostic intégré (1) selon la revendication 1, dans lequel la partie de fixation de cartouche (130) du châssis de base (100) comporte :
un ressort de fixation (131) configuré pour lever et fixer un dessous de la cartouche de diagnostic (500) ;
une saillie de soulèvement (132) configurée pour être adaptée à une portion de la rainure inférieure (560) de la cartouche de diagnostic (500) et fixer celle-ci ; et
un châssis d'ajustement latéral (134) configuré pour fixer la cartouche de diagnostic (500) en insérant un côté de la cartouche de diagnostic (500) dans celui-ci.

10. Lecteur de fluorescence d'immunodiagnostic intégré (1) selon la revendication 2, dans lequel le module de détection de position de référence (400) comporte :
une carte principale de module (410) fixée dans une direction opposée à la partie d'entrée (110) du châssis de base (100) ; et
une partie de capteur d'interruption (420) disposée dans une direction opposée à la partie d'entrée (110) sur la carte principale de module (410), dans lequel la partie de saillie d'interruption (260) est insérée dans la partie de capteur d'interruption (420),
dans lequel une position du module optique (200) est déplacée entre la position de référence où la partie de saillie d'interruption (260) est insérée dans la partie de capteur d'interruption (420) et une position de la partie d'entrée (110).

11. Lecteur de fluorescence d'immunodiagnostic intégré (1) selon la revendication 10, dans lequel le module de détection de position de référence (400) comporte en outre un commutateur de détection de cartouche (430) disposé sous la carte principale de module (410),
dans lequel la cartouche de diagnostic (500) est configurée pour presser le commutateur de détection de cartouche (430) lorsque la cartouche de diagnostic (500) est insérée dans le châssis de base (100) à travers la partie d'entrée (110) et une extrémité de la cartouche de diagnostic (500) atteint une position spécifique, et
dans lequel le commutateur de détection de cartouche (430) génère un signal d'insertion pour la cartouche de diagnostic (500) lorsque la cartouche de diagnostic (500) presse le commutateur de détection de cartouche (430).

12. Lecteur de fluorescence d'immunodiagnostic intégré (1) selon la revendication 1, dans lequel la cartouche de diagnostic (500) comporte la fenêtre de mesure de fluorescence (530) s'étendant dans une direction d'écoulement latéral d'un échantillon et ayant l'écoulement latéral de l'échantillon exposé vers le haut, et un code à barres (510) pour identifier un type d'échantillon,
dans lequel le module optique (200) est configuré pour :
identifier le type d'échantillon en lisant le code à barres (510) en se déplaçant jusqu'à la zone de code à barres,
se déplacer jusqu'à un point de fin (C) de la fenêtre de mesure de fluorescence (530) après avoir détecté un instant de début d'écoulement latéral de l'échantillon à un point de début (B) de la fenêtre de mesure de fluorescence (530), et
calculer un débit de l'écoulement latéral en détectant un temps de fin d'écoulement latéral de l'échantillon au point de fin (C) de la fenêtre de mesure de fluorescence (530).

13. Lecteur de fluorescence d'immunodiagnostic (1) selon la revendication 6 ou 7, dans lequel la cartouche de diagnostic (500) comporte la fenêtre de mesure de fluorescence (530) s'étendant dans une direction d'écoulement latéral d'un échantillon et ayant l'écoulement latéral de l'échantillon exposé vers le haut, et un code à barres (510),
dans lequel le module optique (200) est configuré pour :
identifier le type d'échantillon en lisant le code à barres (510) en déplaçant la source de lumière de code à barres (225) pour rayonner une lumière vers le code à barres (510) ;
se déplacer jusqu'à un point de fin (C) de la fenêtre de mesure de fluorescence (530) après avoir détecté un instant de début d'écoulement latéral de l'échantillon à un point de début (B) de la fenêtre de mesure de fluorescence (530), et
calculer un débit de l'écoulement latéral en détectant un temps de fin d'écoulement latéral de l'échantillon au point de fin (C) de la fenêtre de mesure de fluorescence (530).
